(19) Europäisches Patentamt
European Patent Office
Office européen des brevets

(11) Veröffentlichungsnummer: **0 008 367**
**B1**

(12) # EUROPÄISCHE PATENTSCHRIFT

(45) Veröffentlichungstag der Patentschrift: **10.08.83**

(51) Int. Cl.³: **C 07 D 207/48,** C 07 D 405/12, C 07 D 409/12, A 61 K 31/40

(21) Anmeldenummer: **79102522.4**

(22) Anmeldetag: **18.07.79**

(54) **N-Arylsulfonylpyrrole, ihre Herstellung und diese enthaltende therapeutische Mittel.**

(30) Priorität: **20.07.78 DE 2831850**
**11.04.79 DE 2914615**

(43) Veröffentlichungstag der Anmeldung: **05.03.80 Patentblatt 80/5**

(45) Bekanntmachung des Hinweises auf die Patenterteilung: **10.08.83 Patentblatt 83/32**

(84) Benannte Vertragsstaaten: **AT BE CH DE FR GB IT LU NL SE**

(56) Entgegenhaltungen:
**DE-A-1 964 503**
**DE-A-2 419 970**
**DE-A-2 453 548**
**DE-A-2 518 999**
**DE-A-2 756 783**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Bliesener, Jens-Uwe, Dr. Chem., Silvanerweg 16, D-6705 Deidesheim (DE)**
Erfinder: **Geiss, Karl-Heinz, Dr. Chem., Kirchenstrasse 8, D-6711 Beindersheim (DE)**
Erfinder: **Lenke, Dieter, Dr., Kekuléplatz 1, D-6700 Ludwigshafen (DE)**
Erfinder: **Mueller, Claus D., Dr., Odenwaldring 84, D-6806 Viernheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

N-Arylsulfonylpyrrole, ihre Herstellung und diese enthaltende therapeutische Mittel

Es sind beispielsweise aus den DE-A-2453548, 2518999, 1964503, 2419970 und 2756783 eine grosse Zahl diuretisch wirksamer 5-Sulfamoylbenzoesäuren bekannt. Dabei kann sowohl der Benzolring als auch die Sulfonamidgruppe verschiedenartig substituiert sein.

Gegenstand der vorliegenden Erfindung sind substituierte Benzoesäuren mit einem Sulfonylpyrrolrest, die bisher noch nicht hergestellt worden sind. Die erfindungsgemässen Verbindungen zeichnen sich beispielsweise gegenüber der 3-N-Pyrrolidino-4-phenoxy-5-sulfamoylbenzoesäure (Beispiel 5 der DE-OS 2419970) bei der pharmakologischen Untersuchung der diuretischen Wirkung durch eine überraschenderweise verbesserte Wirksamkeit aus. Auch gegenüber dem im Handel befindlichen Diuretikum Furosemid ergeben sich wesentlich günstigere Eigenschaften, wie weiter unten aufgezeigt wird.

Es wurde gefunden, dass Verbindungen der Formel (1)

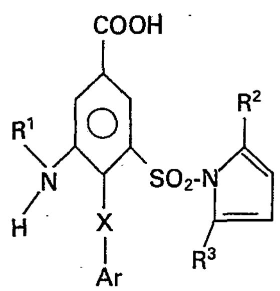 (1)

in der

- $R^1$ einen Alkylrest mit 2 bis 5 C-Atomen, einen Allylrest, einen Benzylrest, einen Furylmethylrest oder einen Thienylmethylrest,
- $R^2$ und $R^3$ Wasserstoffatome oder
- $R^2$ Methyl oder Ethyl und
- $R^3$ Methyl, Ethyl oder n-Propyl,
- X ein Schwefel- oder Sauerstoffatom oder eine >NH-Gruppe und
- Ar einen Phenylrest, der ggf. durch Chlor substituiert ist,

bedeuten, und ihre therapeutisch verwendbaren Ammonium-, Alkalimetall- oder Säureadditionssalze insbesondere als Diuretika wertvolle pharmakologische Eigenschaften aufweisen.

Davon sind besonders bevorzugt solche Verbindungen, in denen

- $R^1$ n-Butyl, Benzyl, 3-Thienylmethyl, 2-Thienylmethyl, 3-Furylmethyl oder 2-Furylmethyl,
- $R^2$ Methyl oder Ethyl,
- $R^3$ Methyl, Ethyl oder n-Propyl,
- X ein Schwefelatom, Sauerstoffatom oder eine >NH-Gruppe und
- Ar einen Phenylrest bedeuten

und ihre therapeutisch verwendbaren Ammonium- und Alkalimetallsalze.

Die Verbindungen der Formel (1) können in an sich üblicher Weise in ihre Ammonium- oder Alkalimetallsalze, insbesondere Natrium- und Kaliumsalze, überführt werden. Gegenstand der Erfindung sind weiterhin auch die physiologisch verträglichen Säureadditionssalze, wie sie in üblicher Weise hergestellt und verwendet werden.

Die Verbindungen der Formel (1) werden hergestellt, indem man

a) eine Verbindung der Formel (2)

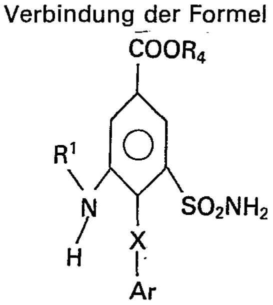 (2)

in der $R^1$, X und Ar die in Anspruch 1 angegebenen Bedeutungen haben und $R^4$ für ein Wasserstoffatom oder einen Alkylrest mit 1 bis 5 C-Atomen steht, mit einer Verbindung der Formel (3)

$$R^2\text{-CO-}CH_2\text{-}CH_2\text{-CO-}R^3$$

(3)

in der $R^2$ und $R^3$ die in Anspruch 1 angegebenen Bedeutungen haben, oder, wenn $R^2$ und $R^3$ Wasserstoffatome sind, zweckmässigerweise mit einer Verbindung der Formel (3a)

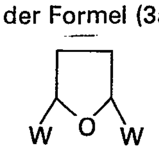

(3a)

in der W für ein Chloratom, einen Alkoxyrest mit 1 bis 5 C-Atomen im Alkyl oder einen Alkanoyloxyrest mit 1 bis 5 C-Atomen im Alkyl steht, gegebenenfalls in einem Lösungsmittel und in Gegenwart einer anorganischen Säure, organischen Carbonsäure oder Sulfonsäure in an sich üblicher Weise umsetzt und gegebenenfalls einen erhaltenen Ester in die Carbonsäure und die erhaltene Verbindung der Formel (1) gegebenenfalls in ein therapeutisch verwendbares Ammonium-, Alkalimetall- oder Säureadditionssalz überführt,

oder

b) – falls $R^1$ im Rahmen der für Formel (1) in Anspruch 1 genannten Bedeutungen eine zum N-Atom alpha-ständige $CH_2$-Gruppe aufweist – eine Acylaminoverbindung der Formel (4)

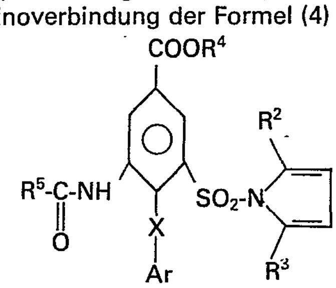 (4)

in der $R^2$, $R^3$, X und Ar die im Anspruch 1 angegebenen Bedeutungen haben, $R^4$ einen Alkylrest mit 1 bis 3 C-Atomen und $R^5$ einen Alkylrest mit 1 bis 4

C-Atomen, Phenyl, einen Furyl- oder einen Thienylrest bedeuten, mit einem Borhydrid in Gegenwart einer Lewis-Säure in an sich bekannter Weise reduziert und den erhaltenen Ester verseift und die erhaltene Verbindung gegebenenfalls in ein therapeutisch verwendbares Ammonium-, Alkalimetall- oder Säureadditionssalz überführt.

In den Ausgangsverbindungen (3a) stellen die Reste W bevorzugt Methoxy, Ethoxy, Acetoxy und Propionyloxy dar. Die besonders bevorzugte Ausgangsverbindung ist das 2,5-Dimethoxytetrahydrofuran.

Die Umsetzung erfolgt vorzugsweise in Eisessig oder wässriger Essigsäure unter Erhitzen bis zum Siedepunkt, wie es beispielsweise von J. W. F. Wasby, K. Chan in Synth. Commun. 3, 303 ff (1973) oder von A. D. Josey und E. L. Jenner in J. Org. Chem., Band 27, S. 2466 bis 2470 (1962) beschrieben wird.

In einer anderen Verfahrensweise werden die Ausgangsverbindungen (2) und (3) oder (3a) in einem unter den Reaktionsbedingungen inerten organischen Lösungsmittel, z. B. einem aromatischen Kohlenwasserstoff, wie Benzol, Toluol, Ethylbenzol, Chlorbenzol, Dichlorbenzol, o-, m-, p-Xylol, Isopropylbenzol, Methylnaphthalin, einem aliphatischen oder cycloaliphatischen Kohlenwasserstoff, wie Ligroin, Petrolether, Heptan, Hexan, Cyclohexan, oder einem entsprechenden Gemisch und in Gegenwart einer katalytischen Menge einer anorganischen Säure, organischen Carbonsäure oder Sulfonsäure, vorzugsweise p-Toluolsulfonsäure, bei Temperaturen von 40 bis 200°C, vorzugsweise von 60 bis 150°C, drucklos oder unter Druck umgesetzt.

Von den genannten Lösungsmitteln sind Cyclohexan und Toluol bevorzugt.

Die erhaltenen Ester lassen sich durch dem Fachmann bekannte Verfahren in die Säure überführen.

Die Ausgangsverbindungen der Formel (2) sind bekannt oder können beispielsweise nach den in den DE-A-1964503 und 2419970 beschriebenen Verfahren hergestellt werden.

Nach dem Verfahren b) können nicht alle erfindungsgemässen Verbindungen gemäss Formel (1) hergestellt werden. Die bevorzugten Bedeutungen für $R^5$ sind Methyl, Ethyl, n-Propyl, n-Butyl, Phenyl, 2- und 3-Thienyl, 2- und 3-Furyl.

Die Reduktion der Carbonylgruppe kann beispielsweise unter den in der DE-A-2453548 beschriebenen Bedingungen erfolgen. Zweckmässigerweise wird sie in einem inerten Lösungsmittel bei Temperaturen von −20 bis 100°C durchgeführt. In der bevorzugten Ausführungsform wird die Reaktion mit Diboran in Gegenwart von Aluminiumchlorid, Titantetrachlorid oder Borfluorid, bzw. seiner Addukte, wie beispielsweise Borfluoridetherat, als Lewis-Säure in einem Ether als Lösungsmittel durchgeführt.

In einer besonders bevorzugten Ausführungsform wird die zu reduzierende Verbindung der Formel (4) in Diethylether, Tetrahydrofuran oder Ethylenglykoldimethylether als Lösungsmittel mit Bortrifluorid bzw. seinem Etherat gelöst und bei 0 bis 40°C durch Zugabe von Natriumborhydrid in fester Form oder als Suspension in einem geeigneten Lösungsmittel Diboran in situ erzeugt und dadurch die Reduktion durchgeführt.

Interessanterweise erfolgt die Reduktion ohne Spaltung der recht labilen $SO_2$-N-Bindung und die Pyrrol-Verbindung erleidet in Gegenwart von Lewis-Säuren keine Nebenreaktionen, wie es u. U. zu erwarten wäre (vgl. Gossauer «Die Chemie der Pyrrole», Springer Verlag 1974, S. 324 ff).

Durch alkalische oder saure Verseifung eines erhaltenen Esters werden die Säuren der Formel (1) in an sich bekannter Weise erhalten. Bevorzugt erfolgt die Hydrolyse in wässriger Lösung mit äquimolekularer Menge an Base, bevorzugt Natronlauge, bei Temperaturen zwischen 20 und 100°C.

Herstellung von Zwischenprodukten für die Ausgangsverbindungen der Formel (2) und (4) und Herstellung der Ausgangsverbindungen der Formel (2) und (4):

4-Halogen-3-halogensulfonyl-5-nitrobenzoesäure bzw. ihre Alkalimetallsalze oder Niedrigalkylester der allgemeinen Formel (5)

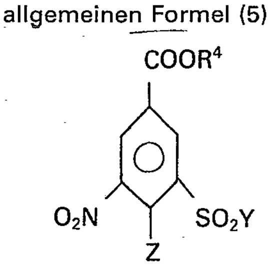

(5)

in der Y und Z jeweils ein Halogenatom, wie Fluor, Chlor oder Brom, bedeuten und $R^4$ für ein Wasserstoffatom, ein Alkalimetallatom oder eine Niedrigalkylgruppe, insbesondere Methyl oder Ethyl, steht, können mit einem Pyrrol der allgemeinen Formel (6),

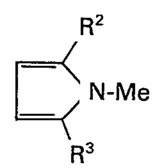

(6)

in der Me ein Metallatom der ersten Hauptgruppe bedeutet und $R^2$ und $R^3$ die für Formel (1) angegebenen Bedeutungen haben, in einem unter den Reaktionsbedingungen inerten Lösungsmittel bei Temperaturen von −20 und 200°C zu den Verbindungen der allgemeinen Formel (7),

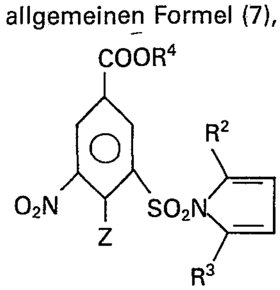

(7)

in der $R^2$ und $R^3$ die für Formel (1) und $R^4$ und Z die für Formel (5) genannten Bedeutungen aufweisen, umgesetzt werden.

Als geeignete Alkalimetalle für Me sind zu nennen:

Lithium, Natrium und Kalium. Geeignete Lösungsmittel sind beispielsweise Dimethylsulfoxid, Dimethylformamid, N-Methylpyrrolidon, Tetrahydrofuran, Diethylether, Ethylenglykoldimethylether, Diethylenglykoldimethylether, Tetramethylethylendiamin und Toluol. Die Pyrrole der Formel (6) können in an sich bekannter Weise, A. Gossauer «Die Chemie der Pyrrole», Springer-Verlag Berlin, 1974, S. 169 f., aus Pyrrol durch Reaktion mit einer Alkalimetallverbindung in einem unter den Reaktionsbedingungen inerten Lösungsmittel, bevorzugt im gleichen Lösungsmittel, das zur Reaktion mit Verbindungen (5) benutzt wird, hergestellt werden. Geeignete Basen sind beispielsweise Alkalimetallalkoholate, Alkalimetallamide, Alkalimetallhydride, Alkyl- oder Arylalkalimetall-Verbindungen und die Alkalimetalle, z. B. Kaliumtertiärbutylat, Natriumamid, Natriumhydrid, Kaliumhydrid, n-Butyllithium, n-Butylnatrium, Natrium und Kalium.

Wie aus der Literatur bekannt ist, E. P. Papadopoulos und N. F. Haider, Tetra. Lett. 1968, 1721, verläuft die Umsetzung von Arylsulfonylchloriden mit Pyrrolkalium nur dann zufriedenstellend, wenn der Benzolkern entweder unsubstituiert ist oder in p-Stellung eine Gruppe mit +M-Effekt, wie z. B. ein Halogenatom oder einen $CH_3O$-Rest, trägt. Durch Einführung einer p-Nitrogruppe sinkt die Ausbeute drastisch auf 26% gegenüber 87% bei Benzolsulfochlorid.

Es ist daher überraschend und nicht vorhersehbar, dass die Umsetzung der Verbindungen der Formel (5), in denen der Benzolring ausser einer Nitrogruppe weitere elektronegative Gruppen trägt, ebenfalls im gewünschten Sinne abläuft.

In der bevorzugten Ausführungsform werden Pyrrole der Formel (6) in einem Ether als Lösungsmittel bei Temperaturen von 0 bis 150°C mit einer Verbindung der Formel (5) umgesetzt. Besonders bevorzugt ist die Reaktion der Pyrrole (6), in denen Me ein Kaliumatom bedeutet, in Tetrahydrofuran bei 0 bis 80°C mit einer Verbindung der Formel (5), in der $R^4$ Methyl oder Ethyl und Y und Z je ein Chloratom bedeuten. Die Pyrrol-Verbindung (6), in der Me Kalium bedeutet, wird nach literaturbekanntem Verfahren aus Pyrrol mit elementarem Kalium als Lösung in Tetrahydrofuran hergestellt und ohne Isolierung weiter umgesetzt.

In der nächsten Stufe wird eine Verbindung der allgemeinen Formel (7) mit einer Verbindung ArXH, in der Ar die für Formel (2) angegebene Bedeutung hat und X ein Sauerstoff-, Schwefelatom oder die Gruppe >NH bedeutet, zu N-Sulfonylpyrrolen der Formel (8)

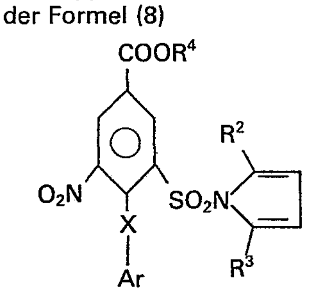

(8)

in der $R^2$, $R^3$ und $R^4$ die für Formel (7), X und Ar die für Formel (1) angegebenen Bedeutungen aufweisen, umgesetzt.

Als Verbindungen der Formel ArXH können beispielsweise verwendet werden: Thiophenol, 2-Chlorthiophenol, 4-Chlorthiophenol, 3,4-Dichlorthiopenol, Phenol, Anilin und analog dem Thiophenol substituierte Phenole und Aniline.

Die Reaktion kann mit oder ohne Lösungsmittel durchgeführt werden. Vorteilhafter ist es, ein Lösungsmittel zu verwenden. Besonders geeignet sind organische Lösungsmittel, wie Ether und tertiäre Amide, insbesondere Tetrahydrofuran, Glykoldimethylether, Dimethylformamid, N-Methylpyrrolidon oder Hexamethylphosphorsäuretriamid. Die Verbindungen ArXH werden als solche in Gegenwart von Basen oder aber in Form ihrer Alkalimetallsalze eingesetzt. Als Basen kommen Alkalihydroxide, -alkoholate, -amide und -hydride zur Anwendung. Besondere Bedeutung haben die Thiophenol- und Phenol-Derivate, die, wie bereits aufgezeigt, durch Chlor substituiert sein können.

Da bekanntlich N-acylierte Pyrrole unter alkalischen Bedingungen sehr leicht gespalten werden, Gossauer, «Die Chemie der Pyrrole», Springer-Verlag, Berlin 1974, S. 324, war es nicht vorherzusehen, dass unter den obigen Bedingungen die N-Sulfonylpyrrol-Gruppe erhalten bleibt. Weiter war es überraschend, dass zum Austausch von Z gegen XAr Temperaturen von über 100°C, wie sie in DE-A-2518999 für die Umsetzung ähnlicher Verbindungen empfohlen wurden, nicht notwendig sind, da die Reaktion bereits unterhalb 100°C mit ausreichender Geschwindigkeit abläuft.

Besonders vorteilhaft wird die Umsetzung einer Verbindung (7) in einem Ether als Lösungsmittel, wie beispielsweise Tetrahydrofuran, bei Temperaturen von 0 bis 80°C durchgeführt.

Hierbei kommen insbesondere die Natrium- oder Kaliumsalze der Verbindungen ArXH zur Anwendung oder die Umsetzungen werden in Gegenwart eines Natrium- oder Kaliumalkoholats, wie Natriummethylat oder Kaliumtertiärbutylats, durchgeführt.

Die Reduktion der Nitrogruppe in einer Verbindung der Formel (8) zu den Verbindungen der Formel (9),

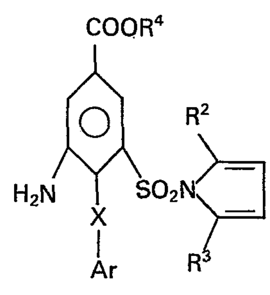

(9)

in denen $R^2$, $R^3$, $R^4$, X und Ar die für Formel (8) angegebenen Bedeutungen haben, erfolgt in an sich bekannter Weise durch katalytische Hydrierung.

Die katalytische Reduktion erfolgt in einem Lösungsmittel in Gegenwart eines Katalysators, wie z. B. Palladium, Platin oder Raney-Nickel auf einem geeigneten Trägermaterial. Als Lösungsmittel werden vorzugsweise organische Lösungsmittel, wie Methanol, Ethanol, Essigester, Tetrahydrofuran, Dioxan oder Dimethylformamid, verwendet. Man hydriert bei Raumtemperatur und Normaldruck oder bei erhöhter Temperatur, gegebenenfalls unter Druck im Autoklaven, wobei die Bedingungen so gewählt werden, dass der Pyrrol-Rest bei der Hydrierung erhalten bleibt.

Aus der Literatur ist bekannt, dass Pyrrole mit elektronegativen Substituenten am Stickstoffatom, wie z. B. eine Benzoylgruppe oder eine Ethoxycarbonylgruppe, leicht und unter milden Bedingungen zu Pyrrolidinen hydriert werden, vgl. J. L. Rainey und H. Adkins J. Amer. Chem. Soc. 61, 1104 (1939). Es war daher überraschend und nicht vorherzusehen, dass die Hydrierung der Verbindungen (8) unter Erhalt des Pyrrolringes zu den gewünschten Verbindungen der Formel (9) führt.

Die erhaltenen Amine der Formel (9) können mit einer Verbindung der allgemeinen Formel $R^5COL$, wobei $R^5$ die für Formel (4) angegebenen Bedeutungen hat und L ein Halogenatom, wie Chlor oder Brom, oder den Rest eines aktivierten Esters oder eines gemischten oder symmetrischen Anhydrids darstellt, wobei im letzteren Fall L die Gruppe O-CO-$R^5$ bedeutet, zu einer Verbindung der Formel (4) umgesetzt werden. Gegebenenfalls wird dabei eine erhaltene Carbonsäure in den Ester übergeführt.

Bevorzugte Acylierungsmittel sind z. B. Acetylchlorid, n-Buttersäurechlorid, n-Buttersäureanhydrid, Propionylchlorid, n-Valeroylchlorid, Benzoylchlorid, 2- bzw. 3-Furancarbonsäurechlorid, 2- bzw. 3-Thiophencarbonsäurechlorid.

Die Acylierung erfolgt in an sich bekannter Weise, es war jedoch nicht vorzusehen, dass die Reaktionen der N-Sulfonylpyrrole (9) mit den Verbindungen $R^5COL$ in guter Ausbeute saubere Produkte vom Typ (4) liefern, da bekanntlich Pyrrole ebenfalls sehr leicht acyliert werden können und erwartungsgemäss schlecht auftrennbare Gemische auftreten sollten (R. A. Jones, G. P. Bean «The Chemistry of Pyrroles», Acad. Press, N. Y., 1977, S. 159/60, J. Chem. Soc., C, 1970, 2563).

Nach einer weiteren Ausführungsform können Verbindungen der allgemeinen Formel (7) nach der Reduktion zu den aromatischen Aminen der allgemeinen Formel (10),

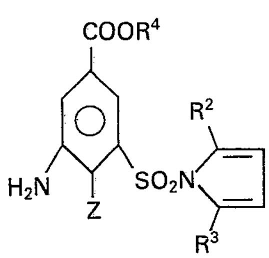 (10)

wobei $R^2$, $R^3$, $R^4$ und Z die in Formel (7) angegebenen Bedeutungen haben mit einer Verbindung der allgemeinen Formel $R^5COL$ zu einer Verbindung der Formel (11)

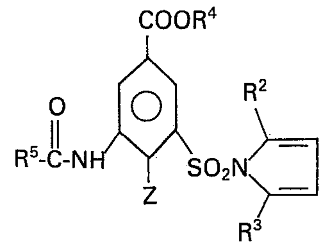 (11)

wobei $R^2$ bis $R^5$ die für Formel (4) und Z die für Formel (7) angegebenen Bedeutungen haben, umgesetzt werden und anschliessend durch Reaktion mit einer Verbindung ArXH in eine Verbindung der Formel (4) umgewandelt werden und eine erhaltene Carbonsäure gegebenenfalls in den Ester überführt.

Weiterhin können Verbindungen der Formel (11), wenn $R^4$ einen Alkylrest darstellt, nach dem oben beschriebenen Verfahren für die Umwandlung von (4) in (1) mit Borhydriden zu Verbindungen der Formel (12),

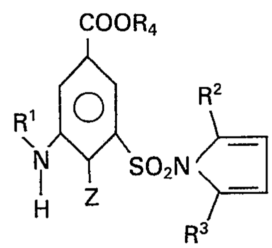 (12)

wobei $R^1$ bis $R^3$ die für Formel (1) und Z die für Formel (7) genannten Bedeutungen haben, reduziert werden.

In einer weiteren Ausführungsform des vorliegenden Verfahrens können Verbindungen der allgemeinen Formel (13),

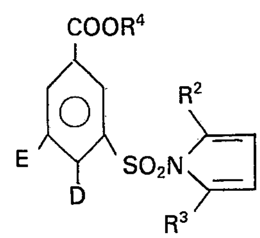 (13)

in denen D für Z oder die Gruppe XAr und E einen der Reste $NO_2$, $R^5CONH$, und, falls D Z bedeutet, die Gruppe $R^1$-NH-, steht, wobei $R^1$ bis $R^3$, X und Ar die oben für Formel (1), Z die oben für Formel (5) und $R^4$ die für Formel (2) angegebenen Bedeutungen haben, durch Umsetzung eines Sulfonamides der allgemeinen Formel (14) (DE-A-1768607, DE-A-1964503, DE-A-2419970, DE-A-2453548)

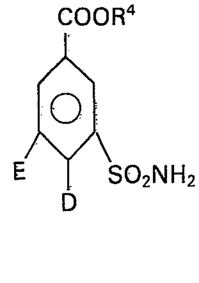 (14)

wobei $R^4$, D und E obige in Formel (13) genannte Bedeutungen aufweisen, nach dem oben beschriebenen Verfahren mit einer Verbindung der Formel (3) oder (3a) hergestellt werden. Diese neuen Verbindungen können als Zwischenstufen in die verschiedenen oben erwähnten Verfahren eingesetzt werden.

Die erfindungsgemässen Verbindungen zeichnen sich durch eine starke diuretische Wirkung aus und sind daher besonders zur Pharmakotherapie von Ödemen verschiedener Genese und des Bluthochdrucks geeignet. Insbesondere können sie daher als Diuretika verwendet werden.

Gegenstand der Erfindung sind auch therapeutische Mittel oder Zubereitungen, die neben üblichen Trägerstoffen oder Verdünnungsmitteln eine Verbindung nach Anspruch 1 als Wirkstoff enthalten. Die therapeutischen Mittel können nach dem Fachmann an sich bekannten Methoden entsprechend der gewünschten Applikationsart erhalten werden.

Die erfindungsgemässen Verbindungen können in üblicher Weise oral oder intravenös verabfolgt werden. Die Dosierung hängt von Alter, Zustand und Gewicht des Patienten sowie von der Applikationsart ab. In der Regel beträgt die tägliche Wirkstoffdosis zwischen 0,1 und 100 mg bei oraler Gabe und zwischen 0,05 und 10,0 mg bei intravenöser Gabe. In besonderen Fällen kann es jedoch erforderlich sein, die Dosen zu steigern.

Die neuen Verbindungen können in den üblichen galenischen Applikationsformen, fest oder flüssig, angewendet werden, wie Tabletten, Kapseln, Pulver, Granulate, Dragées oder Lösungen. Diese werden in üblicher Weise hergestellt. Die Wirkstoffe können dabei mit den gebräuchlichen galenischen Hilfsmitteln, wie Talkum, Gummi arabicum, Saccharose, Lactose, Getreide- oder Maisstärke, Kartoffelmehl, Magnesiumstearat, Alginaten, Gummi tragacanth, Carraghenate, Polyvinylalkohol, Polyvinylpyrrolidon, wässrigen oder nicht wässrigen Trägern, Netzmitteln, Dispergiermitteln, Emulgatoren und/oder Konservierungsmitteln verarbeitet werden (vgl. L. G. Goodman, A. Gilman, The Pharmacological Basis of Therapeutics). Die so erhaltenen Präparate enthalten den Wirkstoff normalerweise in einer Menge von 0,001 bis 99 Gew.-%.

Die Bestimmung der diuretischen Wirkung erfolgte an männlichen Beagle-Hunden im Gewicht von 10 bis 15 kg.

Den Tieren wird 18 h vor Versuchsbeginn das Futter entzogen. 2 h vor Applikation der Prüfsubstanz erhalten sie 20 ml/kg Wasser per os. Die Applikation der Prüfsubstanzen erfolgt per os als Traganth-Suspension. Anschliessend wird im Abstand von 1 h 6mal 4 ml/kg Wasser appliziert.

Die Harnentnahme erfolgt durch Katheterisierung in Abständen von 2 h über einen Zeitraum von 6 h.

Ausser dem Harnvolumen (ml/kg) wird fotometrisch die $Na^+$-, $K^+$- und coulometrisch die $Cl^-$-Ausscheidung (µval/kg) bestimmt.

Zur Bestimmung der akuten Toxizität erhalten Gruppen von 10 weiblichen Mäusen des Stamms NMRI im Gewicht von 20 bis 23 g die Substanzen oral appliziert. Die mittlere letale Dosis (LD 50) wird nach einer Beobachtungszeit von 72 h ermittelt.

Als Vergleichssubstanz diente das bekannte Diureticum Furosemid (4-Chlor-N-(2-furylmethyl)-5-sulfamoyl-anthranilsäure).

Die erfindungsgemässen Verbindungen zeichnen sich durch starke diuretische Wirkung aus. In Tabelle 1 sind die Dosen enthalten, die eine dem Furosemid vergleichbare Erhöhung der $Na^+$- und $H_2O$-Ausscheidung bewirken. Danach sind die Substanzen 2,2mal (Beispiel 8) bis 10mal (Beispiel 23 und 25) wirksamer als Furosemid.

Beim Furosemid steigt die $Na^+$-Ausscheidung zwischen der 2. und 6. h nach der Applikation nicht mehr signifikant an (von 1370 auf 1530 µval/kg). Dagegen wird bei den Substanzen Beispiel 8 und 11 eine deutliche weitere Zunahme der Ausscheidungen sichtbar. Damit weisen beide Substanzen ausser der höheren Wirksamkeit eine längere Wirkungsdauer als Furosemid auf.

Die erfindungsgemässen Verbindungen weisen höhere Werte für die $Na^+/K^+$-Quotienten auf als Furosemid. Das heisst, dass das Verhältnis der pharmakotherapeutisch erwünschten Erhöhung der $Na^+$-Ausscheidung zur unerwünschten Zunahme der $K^+$-Ausscheidung günstiger ist.

Gemessen an den letalen Dosen bei oraler Applikation an der Maus ist die Toxizität der erfindungsgemässen Verbindungen gering (Tabelle 1). Daraus ergibt sich ein sehr grosser Abstand zwischen den diuretisch wirksamen und den letalen Dosen.

Beim Furosemid ist die letale Dosis 2000mal grösser als die diuretisch wirksame, bei Beispiel 8 2060mal, bei Beispiel 11 6840mal, bei Beispiel 29 mehr als 10000mal und bei den Beispielen 23 und 25 mehr als 21500mal grösser als die diuretisch wirksame.

Tabelle 1

Diuretische Wirkung und akute Toxizität
Applikation per os

| Verbindung Beispiel | Dosis mg/kg | Tierzahl | Elektrolyt- und Wasserausscheidung (Hund) 2-Stunden-Werte $NA^+$ µval/kg | $K^+$ µval/kg | $Cl^-$ µval/kg | $H_2O$ ml/kg | $Na^+/K^+$ | | |
|---|---|---|---|---|---|---|---|---|---|
| Kontrolle | – | 60 | 66 | 67 | 81 | 9,3 | 1,0 | | |
| 8 | 0,464 | 6 | 1520 | 298 | 1940 | 27 | 5,1 | | |
| 11 | 0,215 | 6 | 1240 | 333 | 1520 | 23 | 3,7 | | |
| 23 | 0,1 | 6 | 1410 | 295 | 1780 | 26 | 4,8 | | |
| 25 | 0,1 | 6 | 1150 | 271 | 1510 | 22 | 4,2 | | |
| 29 | 0,215 | 6 | 1260 | 227 | 1600 | 19 | 5,6 | | |
| Furosemid | 1,0 | 6 | 1370 | 449 | 1720 | 25 | 3,1 | | |
| Kontrolle | – | 60 | 212 | 184 | 238 | 27 | 1,2 | – | – |
| 8 | 0,464 | 6 | 2930 | 810 | 4200 | 56 | 3,6 | 50 | 957 |
| 11 | 0,215 | 6 | 1940 | 642 | 2440 | 45 | 3,0 | 40 | 1470 |
| 23 | 0,1 | 6 | 1640 | 442 | 2080 | 42 | 3,7 | 10 | >2150[2)] |
| 25 | 0,1 | 6 | 1560 | 473 | 2100 | 40 | 3,3 | 10 | >2150[2)] |
| 29 | 0,215 | 6 | 2050 | 527 | 2760 | 41 | 3,9 | 10 | >2150[1)] |
| Furosemid | 1,0 | 6 | 1530 | 620 | 1890 | 39 | 2,5 | 50 | 2000 |

1) Keine Letalität nach Applikation von 2150 mg/kg
2) Nach Applikation von 2150 mg/kg starb 1 von 10 Tieren

Die nachfolgenden Beispiele erläutern die Erfindung. Die Verbindungen der folgenden Beispiele sind neben den analytischen Daten auch durch spektroskopische Methoden (IR, NMR) in ihrer Struktur gesichert.

I. Die Ausführungsbeispiele 1 bis 22b betreffen Verbindungen der Formel (4), in denen $R^2$ und $R^3$ stets Wasserstoffatome bedeuten.

Allgemeine Arbeitsvorschrift

A) Eine Mischung von 15 ml Eisessig, 1 mMol einer Verbindung der Formel (2) ($R^4$ = H) und 1,5 mMol 2,5-Dimethoxytetrahydrofuran wird unter Rückfluss gekocht. Durch Entnahme von Proben wird die Beendigung der Reaktion dünnschichtchromatografisch überprüft. Das Reaktionsgemisch wird unter vermindertem Druck fast bis zur Trockene eingeengt und der Rückstand auf ca. 20 ml Eiswasser gegeben. Das ausgeschiedene Rohprodukt wird abgesaugt und getrocknet. In den Fällen, bei denen das Produkt in öliger Form anfällt, wird die wässrige Phase mit Essigester extrahiert. Die Essigesterphase wird mit gesättigter Kochsalzlösung gewaschen, über Natriumsulfat getrocknet und unter vermindertem Druck zur Trockene eingeengt. Je nach Reinheit des Rohproduktes wird die erhaltene Verbindung der Formel (1) durch Umkristallisation aus Ethanol oder Essigester/n-Hexan oder durch Säulenchromatographie an Kieselgel mit dem Laufmittel Methylenchlorid/Essigester isoliert. Die Ausbeuten liegen zwischen 40 und 96%.

Die in den folgenden Beispielen 1 bis 12 angegebenen Verbindungen werden nach dieser Arbeitsvorschrift dargestellt.

B) Eine Mischung von 10 mMol einer Verbindung der Formel (2) ($R^4$ = H), 15 mMol 2,5-Dimethoxytetrahydrofuran und 0,25 g p-Toluolsulfonsäure in 150 ml Toluol wird unter Rückfluss am Wasserabscheider gekocht. Durch Entnahme von Proben wird die Beendigung der Reaktion dünnschichtchromatographisch überprüft. Danach wird unter vermindertem Druck zur Trockene eingeengt und je nach Reinheit des Rohproduktes wird die erhaltene Verbindung der Formel (1) durch Umkristallisation aus Ethanol oder Essigester/n-Hexan oder durch Säulenchromatographie an Kieselgel mit dem Laufmittel Methylenchlorid/Essigester isoliert.

Die in den folgenden Beispielen 1 bis 12 angegebenen Verbindungen werden nach dieser Vorschrift praktisch in gleicher Ausbeute erhalten.

Verbindungen der Formel (1) $R^2$, $R^3$ = H)

| Beispiel Nr. | $R^1$ | X | Ar | Fp °C | Analyse C | H | N | O | S | Cl |
|---|---|---|---|---|---|---|---|---|---|---|
| 1 | $C_2H_5$ | S | $C_6H_5$ | 182–183 | ber. 56,7 | 4,5 | 6,9 | 15,9 | 15,9 | – |
| | | | | | gef. 57,0 | 4,7 | 7,0 | 16,4 | 15,5 | – |
| | | | | | $C_{19}H_{18}O_4S_2N_2$ | | M=402,5 | | | |
| 2 | $CH_2CH_2CH_3$ | S | $C_6H_5$ | 181–182 | ber. 57,7 | 4,8 | 6,7 | 15,4 | 15,4 | – |
| | | | | | gef. 57,9 | 4,8 | 6,7 | 15,4 | 15,4 | – |
| | | | | | $C_{20}H_{20}O_4S_2N_2$ | | M=416,5 | | | |
| 3 | $CH_2CH_2{=}CH_2$ | S | $C_6H_5$ | 175–178 | ber. 57,9 | 4,4 | 6,8 | 15,4 | 15,5 | – |
| | | | | | gef. 58,2 | 4,4 | 6,6 | 15,6 | 15,2 | – |
| | | | | | $C_{20}H_{18}O_4S_2N_2$ | | M=414,5 | | | |
| 4 | $CH_2CH_2CH_2CH_3$ | S | $C_6H_5$ | 166–167 | ber. 58,6 | 5,1 | 6,5 | 14,9 | 14,9 | – |
| | | | | | gef. 58,8 | 5,0 | 6,6 | 15,2 | 14,7 | – |
| | | | | | $C_{21}H_{22}O_4N_2S_2$ | | M=431,5 | | | |
| 5 | $CH_2CH_2CH_2CH_3$ | O | $C_6H_5$ | 166–167 | ber. 60,8 | 5,4 | 6,8 | 19,3 | 7,7 | – |
| | | | | | gef. 61,0 | 5,7 | 6,8 | 19,2 | 7,6 | – |
| | | | | | $C_{21}H_{22}O_5N_2S$ | | M=414,5 | | | |
| 6 | $CH_2CH_2CH_2CH_2CH_3$ | S | $C_6H_5$ | 179–182 | ber. 59,4 | 5,4 | 6,3 | 14,4 | 14,4 | – |
| | | | | | gef. 59,7 | 5,5 | 6,5 | 14,2 | 14,1 | – |
| | | | | | $C_{22}H_{24}O_4N_2S_2$ | | M=445,5 | | | |
| 7 | $CH_2C_6H_5$ | S | $C_6H_5$ | 218–220 | ber. 62,1 | 4,3 | 6,0 | 13,8 | 13,8 | – |
| | | | | | gef. 62,1 | 4,4 | 6,1 | 13,6 | 13,4 | – |
| | | | | | $C_{24}H_{20}O_4N_2S_2$ | | M=464,6 | | | |
| 8 | $CH_2C_6H_5$ | O | $C_6H_5$ | 90–91 | ber. 62,6 | 5,4 | 5,2 | 20,8 | 6,0 | – |
| | | | | | gef. 62,7 | 5,0 | 5,3 | 20,3 | 6,0 | – |
| | | | | | $C_{24}H_{20}N_2S$. $CH_3CO_2C_2H_5$ | | M=537,6 | | | |
| 9 | $CH_2C_6H_5$ | S | p-Cl-$C_6H_4$ | 195–200 | ber. 57,8 | 3,8 | 5,6 | 12,8 | 12,8 | 7,1 |
| | | | | | gef. 57,9 | 3,9 | 5,5 | 13,2 | 12,9 | 7,1 |
| | | | | | $C_{24}H_{19}O_4N_2S_2Cl$ | | M=499 | | | |
| 10 | $CH_2$- 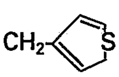 | S | $C_6H_5$ | 192–194 | ber. 56,1 | 3,9 | 5,9 | 13,6 | 20,4 | – |
| | | | | | gef. 56,0 | 3,7 | 5,8 | 13,4 | 20,3 | – |
| | | | | | $C_{22}H_{18}O_4N_2S_3$ | | M=470,6 | | | |
| 11 | $CH_2$ 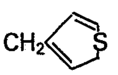 | O | $C_6H_5$ | 174–176 | ber. 58,1 | 4,0 | 6,1 | 17,6 | 14,1 | – |
| | | | | | gef. 58,3 | 4,3 | 6,0 | 17,4 | 13,9 | – |
| | | | | | $C_{22}H_{18}O_5N_2S_2$ | | M=454,5 | | | |
| 12 | $CH_2$ 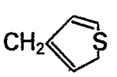 | S | p-Cl-$C_6H_4$ | 212–214 | ber. 52,3 | 3,4 | 5,5 | 12,6 | 19,0 | 7,0 |
| | | | | | gef. 51,9 | 3,5 | 5,4 | 12,4 | 18,7 | 7,5 |
| | | | | | $C_{22}H_{17}O_4N_2S_3Cl$ | | M=505 | | | |

Beispiel 13

3-Benzylamino-4-phenylthio-5-(pyrrol-1-yl-sulfonyl)-benzoesäure

a) 3-Benzylamino-4-phenylthio-5-(pyrrol-1-yl-sulfonyl)-benzoesäuremethylester

Man lässt zu einer Lösung von 2,0 g 3-Benzoylamino-4-phenylthio-5-(pyrrol-1-yl-sulfonyl)-benzoesäuremethylester in 17 ml absolutem Ethylenglykoldimethylether 1 ml Bortrifluoridetherat und anschliessend eine Suspension von 0,24 g Natriumborhydrid in 15 ml absolutem Ethylenglykoldimethylether zutropfen und lässt bei Raumtemperatur rühren. Dann wird mit wenig Wasser überschüssiges Reduktionsmittel zerstört und das Produkt durch Zusatz von 50 ml Wasser ausgefällt. Nach Abfiltrieren und Waschen mit Wasser und Hexan erhält man 1,8 g 3-Benzylamino-4-phenylthio-5-(pyrrol-1-yl-sulfonyl)-benzoesäuremethylester vom Fp. 151–153°C $C_{25}H_{22}N_2O_4S_2$ M= 479

Ausbeute: 95%.

Analyse:

ber.: C 62,7 H 4,6 N 5,8 O 13,3 S 13,4

gef.: C 62,9 H 4,7 N 5,8 O 13,2 S 13,1

b) Hydrolyse des Methylesters

Eine Lösung von 1,0 g 3-Benzylamino-4-phenylthio-5-(pyrrol-1-yl-sulfonyl)-benzoesäuremethylester in 70 ml Ethanol wird mit einer Lösung von 0,083 g NaOH in 30 ml Wasser versetzt und zunächst 4 h bei Raumtemperatur, dann zur Vervollständigung der Reaktion 1 h bei 40°C gerührt. Nach Abziehen des Ethanols im Vakuum wird die wässrige Phase mit verdünnter Salzsäure angesäuert und das Produkt abfiltriert und aus Essigester umkristallisiert. Man erhält 3-Benzylamino-4-phenylthio-5-(pyrrol-1-yl-sulfonyl)-benzoesäure vom Fp. 218–220°C. Ausbeute: 60%.

Analog der Vorschrift gemäss Beispiel 13a werden die Methylester der Beispiele 14a bis 22a hergestellt in Ausbeuten von 80 bis 95% und durch Hydrolyse gemäss der Vorschrift von Beispiel 13b in die erfindungsgemässen Säuren überführt, Beispiele 14b bis 22b, wobei Ausbeuten von 60 bis 95% erhalten werden.

Methylester der Verbindungen gemäss Formel (1) ($R^2$, $R^3$ = H, Ar = $C_6H_5$)

| Beispiel Nummer | $R^1$ | X | Fp °C | C | H | O | N | S |
|---|---|---|---|---|---|---|---|---|
| 14a | $C_6H_5CH_2$- | NH | 158–159 | ber. 65,1<br>gef. 65,0<br>$C_{25}H_{23}O_4N_3S$ | 5,0<br>5,0<br>M=462 | 13,9<br>14,2 | 9,1<br>9,3 | 6,9<br>6,9 |
| 15a | 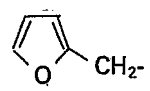 | S | 111–112 | – | | | | |
| 16a | 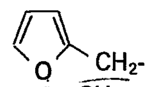 | NH | 129–130 | ber. 61,2<br>gef. 60,9<br>$C_{23}H_{21}O_5N_3S$ | 4,7<br>4,8<br>M=452 | 17,7<br>17,5 | 9,3<br>9,6 | 7,1<br>7,1 |
| 17a | 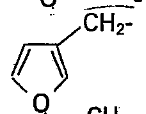 | S | 116–119 | – | | | | |
| 18a | 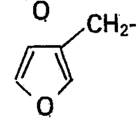 | NH | 138–139 | ber. 61,2<br>gef. 60,9<br>$C_{23}H_{21}O_5N_3S$ | 4,7<br>4,7<br>M=452 | 17,7<br>17,9 | 9,3<br>9,3 | 7,1<br>7,0 |
| 19a | 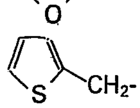 | S | 140–141 | ber. 57,0<br>gef. 57,1<br>$C_{23}H_{20}O_4N_2S_3$ | 4,2<br>4,3<br>M=485 | 13,2<br>13,3 | 5,8<br>5,9 | 19,8<br>19,6 |
| 20a | 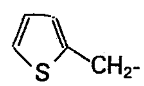 | NH | 152–153 | ber. 59,1<br>gef. 58,8<br>$C_{23}H_{21}O_4N_3S_2$ | 4,5<br>4,5<br>M=468 | 13,7<br>13,7 | 9,0<br>9,3 | 13,7<br>13,5 |
| 21a | 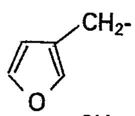 | O | 131–132 | ber. 61,0<br>gef. 60,9<br>$C_{23}H_{20}O_6N_2S$ | 4,4<br>4,4<br>M=452 | 21,2<br>21,0 | 6,2<br>6,1 | 7,0<br>6,9 |
| 22a | 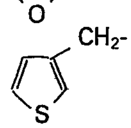 | NH | 149–150 | ber. 59,1<br>gef. 59,0<br>$C_{23}H_{21}O_4N_3S_2$ | 4,5<br>4,7<br>M=468 | 13,7<br>13,5 | 9,0<br>9,2 | 13,7<br>13,5 |
| 14b | $C_6H_5CH_2$- | NH | 228–230 | ber. 64,4<br>gef. 64,6<br>$C_{24}H_{21}O_4N_3S$ | 4,7<br>4,8<br>M=448 | 14,3<br>14,5 | 9,4<br>9,0 | 7,2<br>7,2 |
| 15b | 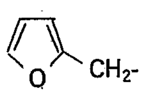 | S | 184–185 | ber. 58,1<br>gef. 57,9<br>$C_{22}H_{18}O_5N_2S_2$ | 4,0<br>4,1<br>M=454,5 | 17,6<br>17,7 | 6,1<br>6,2 | 14,1<br>13,9 |
| 16b | 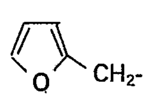 | NH | 186–187 | ber. 60,4<br>gef. 60,6<br>$C_{22}H_{19}O_5N_3S$ | 4,4<br>4,4<br>M=437 | 18,3<br>18,3 | 9,6<br>9,6 | 7,3<br>7,3 |
| 17b | 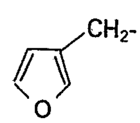 | S | 202–203 | ber. 58,1<br>gef. 57,9<br>$C_{22}H_{18}O_5N_2S_2$ | 4,0<br>4,1<br>M=454,5 | 17,6<br>18,0 | 6,1<br>6,3 | 14,1<br>13,8 |
| 18b | 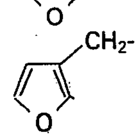 | NH | 195–196 | ber. 60,4<br>gef. 60,3<br>$C_{22}H_{19}O_5N_3S$ | 4,4<br>4,6<br>M=437 | 18,3<br>18,6 | 9,6<br>9,9 | 7,3<br>7,2 |
| 19b | 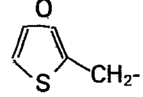 | S | 201–202 | ber. 56,2<br>gef. 56,2<br>$C_{22}H_{18}O_4N_2S_3$ | 3,9<br>3,9<br>M=471 | 13,6<br>13,7 | 6,0<br>6,0 | 20,4<br>19,8 |

| Beispiel Nummer | $R^1$ | X | Fp °C | C | H | O | N | S |
|---|---|---|---|---|---|---|---|---|
| 20b | 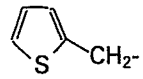 | NH | 205–206 | ber. 58,3 | 4,2 | 14,1 | 9,3 | 14,1 |
| | | | | gef. 58,3 | 4,4 | 13,9 | 9,5 | 13,8 |
| | | | | $C_{22}H_{19}O_4N_3S_2$ | M=454 | | | |
| 21b | 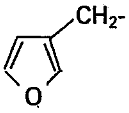 | O | 166–167 | ber. 60,3 | 4,1 | 21,9 | 6,4 | 7,3 |
| | | | | gef. 60,2 | 4,3 | 21,9 | 6,6 | 7,3 |
| | | | | | $C_{22}H_{18}O_6N_2S$ | M=438 | | |
| 22b | 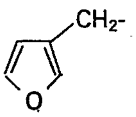 | NH | 201–202 | ber. 58,3 | 4,2 | 14,1 | 9,3 | 14,1 |
| | | | | gef. 58,4 | 4,4 | 14,3 | 9,5 | 14,0 |
| | | | | | $C_{22}H_{19}O_4N_3S_2$ | M=454 | | |

II. Die folgenden Ausführungsbeispiele 23 bis 40 betreffen Verbindungen, in denen $R^2$ und $R^3$ niedere Alkylreste bedeuten.

Allgemeine Arbeitsvorschrift

A') Eine Mischung von 0,02 Mol einer Verbindung der Formel (2) ($R^4$ = Alkyl), 0,15 g p-Toluolsulfonsäure und 0,06 Mol einer Verbindung der Formel (3) in 200 ml trockenem Toluol wird unter Rückfluss am Wasserabscheider gekocht. Durch Entnahme von Proben wird die Beendigung der Reaktion dünnschichtchromatographisch überprüft. Je nach Fortschritt der Reaktion werden nach 10 bis 20 h jeweils 0,03 Mol der Verbindung der Formel (3) erneut zugegeben. Die gesamte Reaktionszeit beträgt zwischen 0,4 und 1,5 Tage. Nachdem die Ausgangsverbindung der Formel (2) dünnschichtchromatographisch nicht mehr nachzuweisen ist, wird die Reaktionsmischung unter vermindertem Druck zur Trockene eingeengt. Je nach Reinheit des Rohproduktes wird die erhaltene Verbindung durch Umkristallisation aus Methanol/Methylenchlorid bzw. Aceton oder durch Säulenchromatographie an Kieselgel mit dem Laufmittel Methylenchlorid isoliert. Die Ausbeuten liegen zwischen 50 und 95%.

B') Hydrolyse des erhaltenen Esters:

Zu einer Lösung von 10 mMol einer nach obiger Vorschrift erhaltenen Verbindung in 250 ml Ethanol wird eine Lösung von 11 mMol Natriumhydroxid in 140 ml Wasser zugegeben. Die Reaktionsmischung wird 3 h unter Rückfluss gekocht. Danach wird der Alkohol abdestilliert und der wässrige Rückstand mit 2n Salzsäure bis pH = 1 angesäuert.

Die ausgefallene Säure wird abgesaugt und getrocknet. Ausbeute: 95 bis 99%.

Die in den folgenden Beispielen 23 bis 40 angegebenen Verbindungen wurden nach diesen Vorschriften hergestellt.

Verbindungen der Formel (1) (Ar = $C_6H_5$)

| Beispiel Nr. | $R^1$ | $R^2$ | $R^3$ | X | Fp °C | Analyse C | H | O | N | S |
|---|---|---|---|---|---|---|---|---|---|---|
| 23 | 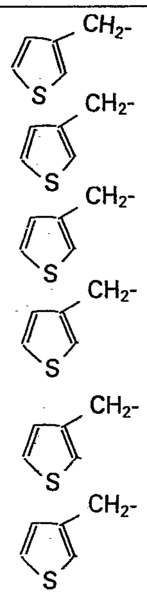 | $C_2H_5$ | $C_2H_5$ | O | 189–190 | ber. 61,2 | 5,1 | 15,7 | 5,5 | 12,6 |
| | | | | | | gef. 61,5 | 5,3 | 15,6 | 5,5 | 12,1 |
| | | | | | | $C_{26}BH_{26}O_5N_2S_2$ | M=511 | | | |
| 24 | | $CH_3$ | $CH_2CH_2CH_3$ | O | 170–173 | ber. 61,2 | 5,1 | 15,7 | 5,5 | 12,6 |
| | | | | | | gef. 60,9 | 5,0 | 15,7 | 5,4 | 12,2 |
| | | | | | | $C_{26}H_{26}O_5N_2S_2$ | M=511 | | | |
| 25 | | $CH_3$ | $C_2H_5$ | O | 200–201 | ber. 60,5 | 4,9 | 16,1 | 5,6 | 12,5 |
| | | | | | | gef. 60,5 | 5,0 | 16,1 | 5,9 | 12,7 |
| | | | | | | $C_{25}H_{24}O_5N_2S_2$ | M=497 | | | |
| 26 | | $CH_3$ | $CH_3$ | S | 187–189 | ber. 57,8 | 4,5 | 12,8 | 5,6 | 19,3 |
| | | | | | | gef. 57,6 | 4,5 | 13,1 | 5,7 | 18,6 |
| | | | | | | $C_{24}H_{22}O_4N_2S_3$ | M=498,6 | | | |
| 27 | | $CH_3$ | $CH_3$ | O | 200–201 | ber. 59,7 | 4,6 | 16,6 | 5,8 | 13,3 |
| | | | | | | gef. 59,7 | 4,6 | 16,5 | 6,0 | 13,2 |
| | | | | | | $C_{24}H_{22}O_5N_2S_2$ | M=483 | | | |
| 28 | | $CH_3$ | $C_2H_5$ | S | 200–202 | ber. 58,6 | 4,7 | 12,5 | 5,5 | 18,6 |
| | | | | | | gef. 59,0 | 4,9 | 12,5 | 5,4 | 18,2 |
| | | | | | | $C_{25}H_{24}O_4N_2S_3$ | M=513 | | | |

| Beispiel Nummer | R¹ | | | X | Fp °C | C | H | O | N | S |
|---|---|---|---|---|---|---|---|---|---|---|
| 29 | 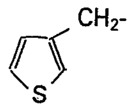 | $CH_3$ | $CH_3$ | NH | 225–226 | ber. 59,9<br>gef. 59,7<br>$C_{24}H_{23}O_4N_3S_2$ | 4,8<br>5,0<br>M=482 | 13,3<br>13,1 | 8,7<br>8,9 | 13,3<br>13,5 |
| 30 | $C_6H_5$-$CH_2$- | $CH_3$ | $CH_3$ | S | 221–223 | ber. 63,4<br>gef. 63,5<br>$C_{26}H_{24}O_4N_2S_2$ | 4,9<br>5,0<br>M=492,6 | 13,0<br>13,0 | 5,7<br>5,7 | 13,0<br>12,0 |
| 31 | $C_6H_5$-$CH_2$- | $CH_3$ | $CH_3$ | O | 159–160 | ber. 65,5<br>gef. 65,6<br>$C_{26}H_{24}O_5N_2S$ | 5,1<br>5,1<br>M=477 | 16,8<br>16,7 | 5,9<br>6,0 | 6,7<br>6,7 |
| 32 | $C_6H_5$-$CH_2$- | $CH_3$ | $CH_3$ | NH | 220–222 | ber. 65,7<br>gef. 65,7<br>$C_{26}H_{25}N_3O_4S$ | 5,3<br>5,2<br>M=476 | 13,5<br>13,4 | 8,8<br>9,0 | 6,7<br>6,6 |
| 33 | $C_6H_5$-$CH_2$- | $CH_3$ | $C_2H_5$ | NH | 178–180 | ber. 66,4<br>gef. 65,7<br>$C_{27}H_{26}O_4N_3S$ | 5,4<br>5,7<br>M=489 | 13,1<br>13,3 | 8,6<br>8,6 | 6,6<br>6,2 |
| 34 | $C_6H_5$-$CH_2$- | $CH_3$ | $C_2H_5$ | S | 194–196 | ber. 64,0<br>gef. 64,1<br>$C_{27}H_{26}O_4N_2S_2$ | 5,2<br>5,5<br>M=507 | 12,6<br>12,7 | 5,5<br>5,6 | 12,7<br>12,3 |
| 35 | $CH_2CH_2CH_2CH_3$ | $CH_3$ | $CH_3$ | O | 160–162 | ber. 62,4<br>gef. 62,4<br>$C_{23}H_{26}O_5N_2S$ | 5,9<br>5,8<br>M=443 | 18,1<br>18,2 | 6,3<br>6,3 | 7,2<br>7,2 |
| 36 | $CH_2CH_2CH_2CH_3$ | $CH_3$ | $CH_3$ | S | 146 | ber. 60,2<br>gef. 59,4<br>$C_{23}H_{26}O_4N_2S_2$ | 5,7<br>5,8<br>M=459 | 14,0<br>15,0 | 6,1<br>6,1 | 14,0<br>13,5 |
| 37 | 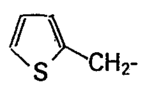 | $CH_3$ | $CH_3$ | S | 201 | ber. 57,8<br>gef. 57,6<br>$C_{24}H_{22}O_4N_2S_3$ | 4,5<br>4,6<br>M=499 | 12,8<br>13,1 | 5,6<br>5,7 | 19,3<br>18,6 |
| 38 | 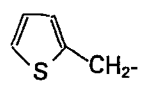 | $CH_3$ | $CH_3$ | O | 184–186 | ber. 59,7<br>gef. 59,5<br>$C_{24}H_{22}O_5N_2S_2$ | 4,6<br>4,5<br>M=483 | 16,6<br>16,4 | 5,8<br>5,6 | 13,3<br>13,6 |
| 39 | 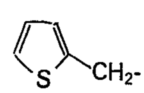 | $CH_3$ | $CH_3$ | NH | 214–216 | ber. 59,8<br>gef. 59,7<br>$C_{24}H_{23}O_4N_3S_2$ | 4,8<br>4,8<br>M=482 | 13,8<br>13,2 | 8,7<br>8,8 | 13,3<br>13,4 |
| 40 | 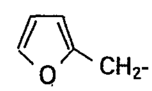 | $CH_3$ | $CH_3$ | NH | 211–213 | ber. 61,9<br>gef. 61,7<br>$C_{24}H_{23}O_5N_3S$ | 5,0<br>5,1<br>M=465,5 | 17,2<br>17,3 | 9,0<br>9,2 | 6,0<br>6,7 |

III. Herstellung von Zwischenprodukten und Ausgangsverbindungen

Beispiel 41

3-Benzoylamino-4-phenylthio-5-(pyrrol-1-yl-sulfonyl)-benzoesäuremethylester

a) 4-Chlor-5-chlorsulfonyl-3-nitro-benzoesäure-methylester

Formel (5): Y, Z = Cl, $R^4$ = $CH_3$

50 g 4-Chlor-5-chlorsulfonyl-3-nitro-benzoesäure [J. Med. Chem. 13, 1971, (1970)] wird in 600 ml halbkonzentrierter methanolischer HCl-Lösung über Nacht bei Raumtemperatur gerührt. Nach Einengen im Vakuum auf 100 ml wird das ausgefallene Produkt abfiltriert und mehrmals mit Ether gewaschen. Man erhält 4-Chlor-5-chlorsulfonyl-3-nitro-benzoesäuremethylester vom Fp.: 92–94°C. Ausbeute: 78%.

$C_8H_5Cl_2NO_6S$ M = 314

Analyse:

ber.: C 30,6 H 1,6 Cl 22,5 N 4,4 O 30,6 S 10,2

gef.: C 30,7 H 1,9 Cl 22,2 N 4,4 O 30,9 S 10,0

b) 4-Chlor-3-nitro-5-(pyrrol-1-yl-sulfonyl)-benzoesäuremethylester

Formel (7): Z = Cl; $R^4$ = $CH_3$; $R^2$, $R^3$ = H

Zu 1 l absolutem Tetrahydrofuran (THF) werden zunächst 10 g metallisches Kalium und anschliessend eine Lösung von 21 ml Pyrrol in 50 ml THF zugegeben. Man kocht bis zum Verschwinden des Kaliums am Rückfluss. Nach Abkühlen auf Raumtemperatur lässt man in 30 bis 60 min eine Lösung von 62 g 4-Chlor-5-chlorsulfonyl-3-nitro-benzoesäuremethylester in 500 ml THF zutropfen und über Nacht bei Raumtemperatur rühren. Anschliessend wird das Lösungsmittel bei 30 bis 35°C im Vakuum grösstenteils abgezogen, der Rückstand mit Wasser versetzt und zweimal mit Essigester extrahiert. Nach dem Trocknen der organischen Phase über Natriumsulfat wird die organische Lösung mit Aktivkohle aufgekocht und filtriert. Nach Einengen im Vakuum fällt das Produkt aus. Man erhält nach Filtration 4-Chlor-3-nitro-5-(pyrrol-1-yl-sulfonyl)-benzoesäuremethylester vom Fp.: 136–137°C
Ausbeute: 70%
$C_{12}H_9ClN_2O_6S$ M = 345
Analyse:
ber.: C 41,8 H 2,6 Cl 10,2 N 8,1 O 27,8 S 9,3
gef.: C 42,0 H 2,8 Cl 10,0 N 8,3 O 27,4 S 9,1

c) 3-Nitro-4-phenylthio-5-pyrrol-1-yl-sulfonyl-benzoesäuremethylester
Formel (8): $R^4 = CH_3$; X = S; Ar = $C_6H_5$; $R^2$, $R^3$ = H

Eine Suspension von 13 g Natriummethylat in 440 ml absolutem THF wird unter Rühren bei Raumtemperatur in einer $N_2$-Atmosphäre mit einer Lösung von 24,5 ml Thiophenol in 100 ml wasserfreiem THF versetzt. Anschliessend werden 72 g 4-Chlor-3-nitro-5-(pyrrol-1-yl-sulfonyl)-benzoesäuremethylester in 220 ml absolutem THF zugetropft und bei Raumtemperatur bis zum dünnschichtchromatographisch bestimmten Ende der Reaktion gerührt. Nach Entfernen des Lösungsmittels am Rotationsverdampfer wird der Rückstand mehrmals mit Hexan ausgezogen und anschliessend aus Essigester/Methanol umkristallisiert. Man erhält 3-Nitro-4-phenylthio-5-(pyrrol-1-yl-sulfonyl)-benzoesäuremethylester vom Fp.: 153–154°C.
Ausbeute: 45%
$C_{18}H_{14}N_2O_6S_2$ M = 418
Analyse:
ber.: C 51,6 H 3,3 N 6,6 O 22,9 S 15,3
gef.: C 51,7 H 3,6 N 6,8 O 22,7 S 14,9

d) 3-Amino-4-phenylthio-5-(pyrrol-1-yl-sulfonyl)-benzoesäuremethylester
Formel (9): $R^4 = CH_3$; X = S; Ar = $C_6H_5$; $R^2$, $R^3$ = H

20 g 3-Nitro-4-phenylthio-5-(pyrrol-1-yl-sulfonyl)-benzoesäuremethylester werden in 1 l Essigester in Gegenwart von 2 g Palladium/Aktivkohle zunächst bei Raumtemperatur dann bei 40 bis 50°C hydriert. Nach Abfiltrieren des Kontaktes und Einengen des Filtrats im Vakuum wird der Rückstand mit Methylenchlorid über eine Kieselgelsäule chromatographiert. Die produkthaltigen Fraktionen werden vom Lösungsmittel befreit und der Rückstand aus Methanol umkristallisiert. Man erhält 3-Amino-4-phenylthio-5-(pyrrol-1-yl-sulfonyl)-benzoesäuremethylester vom Fp.: 118–119°C
Ausbeute: 80%
$C_{18}H_{16}N_2O_4S_2$ M = 388,5
Analyse:
ber.: C 55,6 H 4,1 N 7,2 O 16,5 S 16,5
gef.: C 55,7 H 3,9 N 7,2 O 16,8 S 16,3

e) 3-Benzoylamino-4-phenylthio-5-(pyrrol-1-yl-sulfonyl)-benzoesäuremethylester
Formel (4): $R^2$, $R^3$ = H; $R^4 = CH_3$; X = S; Ar = $C_6H_5$; $R^5 = C_6H_5$

5 g 3-Amino-4-phenylthio-5-(pyrrol-1-yl-sulfonyl)-benzoesäuremethylester, gelöst in 25 ml wasserfreiem Dioxan, werden mit 1,4 ml Pyridin und einer Lösung von 7,2 g Benzoylchlorid in 30 ml Aceton versetzt und bei Raumtemperatur, später bei 40°C gerührt. Das Lösungsmittel wird im Vakuum abgezogen, der Rückstand in Methylenchlorid aufgenommen und mit verdünnter Natriumhydrogencarbonatlösung gewaschen. Nach Trocknen über Natriumsulfat wird das Lösungsmittel im Rotationsverdampfer abgezogen und aus Methanol/Methylenchlorid umkristallisiert. Man erhält 3-Benzoylamino-4-phenylthio-5-(pyrrol-1-yl-sulfonyl)-benzoesäuremethylester vom Fp.: 210–211°C.
Ausbeute: 59%
$C_{25}H_{20}N_2O_5S_2$ M = 492,6
Analyse:
ber.: C 61,0 H 4,1 N 5,7 O 16,2 S 13,0
gef.: C 61,2 H 4,4 N 5,8 O 16,3 S 12,8

Beispiele 42 bis 46

Nach der Vorschrift e) in Beispiel 41 werden die folgenden Verbindungen der allgemeinen Formel (4) aus den Verbindungen der allgemeinen Formel (9) ($R^2$, $R^3$ = H; $R^4 = CH_3$; Ar = $C_6H_5$) hergestellt, wobei Ausbeuten von 55–93% erhalten werden.

Verbindungen der Formel (4) ($R^2$, $R^3$ = H; $R^4 = CH_3$; Ar = $C_6H_5$)

| Beispiel Nr. | $R^5$ | X | Fp °C | Analyse C | H | O | N | S |
|---|---|---|---|---|---|---|---|---|
| 42 | $C_6H_5$ | NH | 191–192 | ber. 63,1 | 4,5 | 16,8 | 8,8 | 6,7 |
| | | | | gef. 63,0 | 4,5 | 16,7 | 8,9 | 6,7 |
| | | | | $C_{25}H_{21}O_5N_3S$ | M=476 | | | |
| 43 | 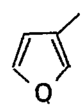 | S | 165–167 | ber. 57,2 | 3,7 | 19,9 | 5,8 | 13,3 |
| | | | | gef. 57,5 | 3,8 | 19,6 | 5,7 | 13,1 |
| | | | | $C_{23}H_{18}O_6N_2S_2$ | M=482,5 | | | |
| 44 | 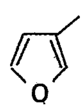 | NH | 182–183 | ber. 59,3 | 4,1 | 20,6 | 9,0 | 6,9 |
| | | | | gef. 59,1 | 4,2 | 20,1 | 9,2 | 6,9 |
| | | | | $C_{23}H_{19}O_6N_3S$ | M=465 | | | |

| Beispiel Nr. | $R^5$ | X | Fp ° C | C | Analyse H | O | N | S |
|---|---|---|---|---|---|---|---|---|
| 45 | 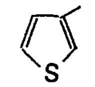 | NH | 193–194 | ber. 57,4 | 4,0 | 16,6 | 8,7 | 13,3 |
| | | | | gef. 57,0 | 4,1 | 16,8 | 8,9 | 13,1 |
| | | | | $C_{23}H_{19}O_5N_3S_2$ | M=482 | | | |
| 46 | 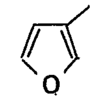 | O | 224–225 | ber. 54,8 | 3,9 | 26,9 | 6,7 | 7,7 |
| | | | | gef. 54,4 | 3,9 | 26,8 | 6,8 | 7,6 |
| | | | | $C_{19}H_{16}O_7N_2S$ | M=416 | | | |

Beispiel 47
3-(2-Furfuroylamino)-4-phenylthio-5-(pyrrol-1-yl-sulfonyl)-benzoesäuremethylester

Formel (4): $R^2$, $R^3$ = H; $R^4$ = $CH_3$; X = S; Ar = $C_6H_5$; $R^5$ – 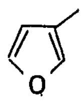

Zu einer Lösung von 5 g 3-Amino-4-phenylthio-5-(pyrrol-1-yl-sulfonyl)-benzoesäuremethylester und 1,4 ml N,N-Dimethylanilin in 25 ml wasserfreiem Dioxan werden 3,4 g 2-Furfuroylchlorid, gelöst in 30 ml Dioxan, zugetropft. Nach 3 bis 8 h Rühren bei Raumtemperatur wird im Vakuum eingeengt, der Rückstand in Methylenchlorid gelöst, die organische Phase mit Wasser und verdünnter Natriumhydrogencarbonatlösung gewaschen, über Natriumsulfat getrocknet und im Rotationsverdampfer vom Lösungsmittel befreit. Nach Umkristallisation aus Methylenchlorid/Methanol erhält man 5 g 3-(2-Furfuroylamino)-4-phenylthio-5-(pyrrol-1-yl-sulfonyl)-benzoesäuremethylester vom Fp.: 165–167°C.
Ausbeute: 87%.

Beispiele 48 bis 50

Analog der Vorschrift aus Beispiel 47 werden aus den Verbindungen der allgemeinen Formel (9) ($R^2$, $R^3$ = H; $R^4$ = $CH_3$, Ar = $C_6H_5$) die folgenden Verbindungen der Formel (4) in Ausbeuten von 75–96% erhalten.

Verbindungen der Formel (4) ($R^2$, $R^3$ = H; $R^4$ = $CH_3$; Ar = $C_6H_5$)

| Beispiel Nr. | $R^5$ | X | Fp ° C | C | Analyse H | O | N | S |
|---|---|---|---|---|---|---|---|---|
| 48 | 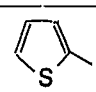 | S | 196–197 | ber. 55,4 | 3,6 | 16,0 | 5,6 | 19,3 |
| | | | | gef. 55,3 | 3,7 | 15,8 | 5,6 | 18,9 |
| | | | | $C_{23}H_{18}N_2O_5S_2$ | M=499 | | | |
| 49 | 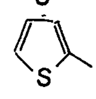 | NH | 185–186 | ber. 57,4 | 4,0 | 16,6 | 8,7 | 13,3 |
| | | | | gef. 57,3 | 3,9 | 16,6 | 9,0 | 13,2 |
| | | | | $C_{23}H_{19}O_5N_3S_2$ | M=482 | | | |
| 50 | 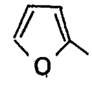 | NH | 160–161 | ber. 59,3 | 4,1 | 20,6 | 9,0 | 6,9 |
| | | | | gef. 59,0 | 4,1 | 20,3 | 9,1 | 6,8 |
| | | | | $C_{23}H_{19}N_3O_6S$ | M=465,6 | | | |

Beispiel 51
3-Benzoylamino-4-phenylthio-5-(pyrrol-1-yl-sulfonyl)-benzoesäuremethylester

a) 3-Amino-4-chlor-5-(pyrrol-1-yl-sulfonyl)-benzoesäuremethylester

Formel (10): $R^4$ = $CH_3$, Z = Cl, $R^2$, $R^3$ = H

Eine Lösung von 55 g 4-Chlor-3-nitro-5-(pyrrol-1-yl-sulfonyl)-benzoesäuremethylester (s. Beispiel 41b) in 800 ml Essigester wird in Gegenwart von 5,0 g Palladium/Aktivkohle (10% Pd) bei 20 bis 40°C bis zum Ende der Wasserstoffaufnahme hydriert (Dauer ca. 7 h). Nach Abtrennen des Katalysators wird das Filtrat vom Lösungsmittel befreit und der Rückstand in Essigester/Methanol (9/1) umkristallisiert. Man erhält den 3-Amino-4-chlor-5-(pyrrol-1-yl-sulfonyl)-benzoesäuremethylester vom Fp.: 178–181°C.
Ausbeute: 70%.
$C_{12}H_{11}N_2O_4SCl$ M = 314,75
Analyse:
ber.: C 45,8 H 3,5 N 8,9 O 20,3 S 10,2 Cl 11,3
gef.: C 45,3 H 3,4 N 8,9 O 20,8 S 9,9 Cl 11,2

b) 3-Benzoylamino-4-chlor-5-(pyrrol-1-yl-sulfonyl)-benzoesäuremethylester

Formel (11): $R^4$ = $CH_3$; Z = Cl; $R^5$ = $C_6H_5$; $R^2$, $R^3$ = H.

Eine Lösung von 10,0 g 3-Amino-4-chlor-5-(pyrrol-1-yl-sulfonyl)-benzoesäuremethylester in 64 ml wasserfreiem Dioxan wird mit 3,2 ml Pyridin

versetzt, anschliessend wird bei Raumtemperatur eine Lösung von 8,9 g Benzoylchlorid in 64 ml Aceton zugetropft. Man lässt über Nacht bei Raumtemperatur rühren, engt im Vakuum ein und nimmt den Rückstand in Essigester auf. Nach zweimaligem Waschen mit Wasser wird die organische Phase über Natriumsulfat getrocknet und im Rotationsverdampfer vom Lösungsmittel befreit. Nach Umkristallisieren aus Methanol erhält man 6,4 g 3-Benzoyl-amino-4-chlor-5-(pyrrol-1-yl-sulfonyl)-benzoesäuremethylester vom Fp.: 181–182°C.
Ausbeute: 61%.
$C_{19}H_{15}ClN_2O_5S$ M = 418,9
Analyse:
ber.: C 54,4 H 3,6 Cl 8,5 N 6,7 S 7,7
gef.: C 54,3 H 3,4 Cl 8,5 N 7,0 S 7,7

c) 3-Benzoylamino-4-phenylthio-5-(pyrrol-1-yl-sulfonyl)-benzoesäuremethylester

Formel (4): ($R^2$, $R^3$ = H, $R^4$ = $CH_3$, X = S; $R^5$, Ar = $C_6H_5$) wird nach Vorschrift c) Beispiel 41 aus 87,4 g der oben erhaltenen Verbindung (Beispiel 51b) hergestellt. Das Produkt wird aus Methanol/Methylenchlorid umkristallisiert. Fp.: 210–211°C.
Ausbeute: 93%.

Die Beispiele 52 und 53 werden analog zu der bei Beispiel 51a) angegebenen Vorschrift, ausgehend von Verbindungen der Formel (13) ($R^2$ bis $R^4$ = H, E = $NO_2$) durch katalytische Hydrierung, erhalten:
Formel (13): $R_2$ bis $R^4$ = H, E = $NH_2$

| Beispiel Nr. | D | Fp °C |
|---|---|---|
| 52 | $SC_6H_5$ | 212–216 |
| 53 | $NHC_6H_5$ | 282–284 |

Beispiel 54
3-Benzylamino-4-chlor-5-(pyrrol-1-yl-sulfonyl)-benzoesäuremethylester
Formel (12): $R^4$ = $CH_3$; Z = Cl; $R^2$, $R^3$ = H; $R^1$ = $C_6H_5$-$CH_2$-

Zu einer Lösung von 1,0 g 3-Benzoylamino-4-chlor-5-(pyrrol-1-yl-sulfonyl)-benzoesäuremethylester in 10 ml trockenem Diglyme und 0,6 ml $BF_3$-Etherat wird bei Raumtemperatur eine Lösung von 0,14 g $NaBH_4$ in 5 ml trockenem Diglyme zugetropft. Nach 1 h wird der $NaBH_4$-Überschuss mit wenig $H_2O$ zerstört, der Niederschlag abfiltriert und das Filtrat unter Kühlung mit ca. 20 ml Wasser versetzt. Das ausgefallene Produkt wird abgesaugt und einmal mit Wasser, dann mit n-Hexan gewaschen. Nach Umkristallisieren aus Methanol erhält man sauberes Produkt vom Fp.: 143–145°C.
Ausbeute: 90%.
$C_{19}H_{17}N_2O_4SCl$ M = 404,87
Analyse:
ber.: C 56,4 H 4,2 N 6,9 O 15,8 S 7,9 Cl 8,7
gef.: C 56,4 H 4,4 N 7,0 O 16,2 S 7,9 Cl 8,7
Die folgenden Beispiele 55 bis 61 werden durch Umsetzung von einer Verbindung der Formel (14) nach der oben angegebenen allgemeinen Arbeitsvorschrift A bzw. B in Ausbeuten von 50 bis 90% erhalten.

Verbindungen der Formel (13) ($R^2$, $R^3$ = H)

| Beispiel Nr. | $R^4$ | D | E | Allgem. Arbeitsvorschrift | Fp °C |
|---|---|---|---|---|---|
| 54 | H | Cl | $NO_2$ | A | 223–225 |
| 55 | H | Cl | $NO_2$ | B | 223–225 |
| 56 | $CH_3$ | Cl | $NO_2$ | A | 138–140 |
| 57 | H | $SC_6H_5$ | $NO_2$ | B | 241–243 |
| 58 | H | $NHC_6H_5$ | $NO_2$ | B | 264–267 |
| 59 | $CH_3$ | Cl | $C_6H_5CONH$ | A | 182–183 |
| 60 | H | $OC_6H_5$ | $NO_2$ | A | 180–182 |
| 61 | $CH_3$ | $OC_6H_5$ | 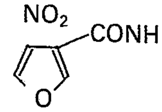 | A | 189–190 |

Die Beispiele 62 bis 66 werden nach der folgenden allgemeinen Arbeitsvorschrift C erhalten, wobei bei den Beispielen 62 bis 64 von einer Verbindung der allgemeinen Formel (13) ($R_2$ bis $R^4$ = H) und bei den Beispielen 65 und 66 von einer Verbindung der allgemeinen Formel (9) ($R^2$ bis $R^4$ = H) ausgegangen wird.

Allgemeine Vorschrift C) für die Herstellung von Methylestern:

C) 0,2 Mol der Carbonsäure und 800 ml halbkonzentrierte methanolische Salzsäure werden ca. 24h lang auf 50°C ehitzt. Das Ende der Reaktion wird dünnschichtchromatographisch überprüft.

Danach wird unter vermindertem Druck zur Trokkene eingeengt und durch Umkristallisation aus Methanol gereinigt.

Ausbeute: 80–95%.

Verbindungen der Formel (13): $R^2$, $R^3$ = H; $R^4$ = $CH_3$

| Bspl. Nr. | E | D | Fp °C |
|---|---|---|---|
| 62 | $NO_2$ | Cl | 138–140 |
| 63 | $NO_2$ | $SC_6H_5$ | 154–157 |
| 64 | $NO_2$ | $NHC_6H_5$ | 135–136 |

Verbindungen der Formel (9): $R^2$, $R^3$ = H; $R^4$ = $CH_3$; Ar = $C_6H_5$

| Beispiel Nr. | X | Fp °C |
|---|---|---|
| 65 | S | 118–119 |
| 66 | NH | 144–146 |

IV. Beispiele für Zubereitungen

Beispiel 1

Auf einer Tablettenpresse werden in üblicher Weise Tabletten folgender Zusammensetzung gepresst:

| | | |
|---|---|---|
| 30 | mg | 3-N-Benzylamino-4-phenoxy-5-[(2,5-dimethyl-pyrrol)-1-yl-sulfonyl]-benzoesäure |
| 150 | mg | Maisstärke |
| 13,50 | mg | Gelatine |
| 45 | mg | Milchzucker |
| 22,5 | mg | Talk |
| 2,25 | mg | Aerosil[R] (chemisch reine Kieselsäure in submikroskopisch feiner Verteilung) |
| 6,75 | mg | Kartoffelstärke (als 6%iger Kleister) |

Beispiel 2

In üblicher Weise werden Dragées folgender Zusammensetzung hergestellt:

| | | |
|---|---|---|
| 20 | mg | 3-N-Benzylamino-4-phenoxy-5-[(2,5-dimethylpyrrol)-1-yl-sulfonyl]-benzoesäure |
| 170 | mg | Kernmasse |
| 160 | mg | Verzuckerungsmasse |

Die Kernmasse besteht aus 9 Teilen Maisstärke, 3 Teilen Milchzucker und 1 Teil Luviskol® VA 64 (Vinylpyrrolidon-Vinylacetat-Mischpolymerisat 60:40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragées werden anschliessend mit einem magensaftresistenten Überzug versehen.

Beispiel 3

10,0 g Na-Salz der 3-N-Benzylamino-4-phenoxy-5-[(2,5-dimethyl-pyrrol)-1-yl-sulfonyl]-benzoesäure werden in 5 l Wasser gelöst. Die Lösung wird mit 0,1n Natriumacetat auf pH 3,5 eingestellt und mit Kochsalz isotonisch eingestellt. Danach wird sie in 2 ml fassende Ampullen steril abgefüllt.

Beispiel 4

10,0 g Na-Salz der 3-N-Benzylamino-4-phenoxy-5-[(2,5-dimethyl-pyrrol)-1-yl-sulfonyl]-benzoesäure werden in 50 l Wasser gelöst, mit Glucose isotonisch eingestellt und auf pH 7,0 neutralisiert. Dann wird sie steril in 200 ml Infusionsbeutel abgefüllt.

**Patentansprüche**

1. Verbindungen der Formel (1)

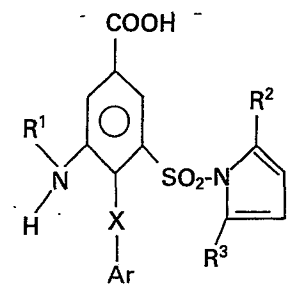

(1)

in der

- $R^1$ einen Alkylrest mit 2 bis 5 C-Atomen, einen Allylrest, einen Benzylrest, einen Furylmethylrest oder einen Thienylmethylrest,
- $R^2$ und $R^3$ Wasserstoffatome oder
- $R^2$ Methyl oder Ethyl und
- $R^3$ Methyl, Ethyl oder n-Propyl,
- X ein Schwefel- oder Sauerstoffatom oder eine NH-Gruppe und
- Ar einen Phenylrest, der ggf. durch Chlor substituiert ist,

bedeuten, und ihre therapeutisch verwendbaren Ammonium-, Alkalimetall- oder Säureadditionssalze.

2. Verbindungen nach Anspruch 1, in denen

- $R^1$ n-Butyl, Benzyl, 3-Thienylmethyl, 2-Thienylmethyl, 3-Furylmethyl oder 2-Furylmethyl
- $R^2$ Methyl oder Ethyl,
- $R^3$ Methyl, Ethyl oder n-Propyl,
- X ein Schwefelatom, Sauerstoffatom oder eine NH-Gruppe und
- Ar einen Phenylrest bedeuten

und ihre therapeutisch verwendbaren Ammonium- und Alkalimetallsalze.

3. Verbindung nach Anspruch 1, in der $R^1$ 3-Thienylmethyl, $R^2$ und $R^3$ Ethyl, X ein Sauerstoffatom und Ar Phenyl bedeuten.

4. Verbindung nach Anspruch 1, in der $R^1$ 3-Thienylmethyl, $R^2$ und $R^3$ Methyl, X eine NH-Gruppe und Ar Phenyl bedeuten.

5. Verbindung nach Anspruch 1, in der $R^1$ Benzyl, $R^2$ und $R^3$ Methyl, X ein Sauerstoffatom und Ar Phenyl bedeuten.

6. Verbindung nach Anspruch 1, in der $R^1$ 2-Thienylmethyl, $R^2$ und $R^3$ Methyl, X ein Sauerstoffatom und Ar Phenyl bedeuten.

7. Verbindung nach Anspruch 1, in der $R^1$ 3-Thienylmethyl, $R^2$ Methyl, $R^3$ Ethyl, X ein Schwefelatom und Ar Phenyl bedeuten.

8. Verbindung nach Anspruch 1, in der $R^1$ 3-Thienylmethyl, $R^2$ Methyl, $R^3$ Ethyl, X ein Sauerstoffatom und Ar Phenyl bedeuten.

9. Verfahren zur Herstellung der Verbindungen nach Anspruch 1, dadurch gekennzeichnet, dass man

a) eine Verbindung der Formel (2)

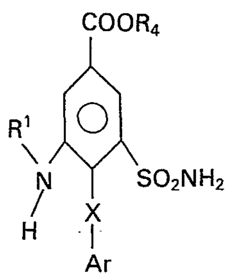

(2)

in der $R^1$, X und Ar die in Anspruch 1 angegebenen Bedeutungen haben und $R^4$ für ein Wasserstoffatom oder einen Alkylrest mit 1 bis 5 C-Atomen steht, mit einer Verbindung der Formel (3)

$$R^2\text{-CO-CH}_2\text{-CH}_2\text{-CO-}R^3$$

(3)

in der $R^2$ und $R^3$ die in Anspruch 1 angegebenen Bedeutungen haben, oder, wenn $R^2$ und $R^3$ Wasserstoffatome sind, zweckmässigerweise mit einer Verbindung der Formel (3a)

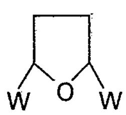

(3a)

in der W für ein Chloratom, einen Alkoxyrest mit 1 bis 5 C-Atomen im Alkyl oder einen Alkanoyloxyrest mit 1 bis 5 C-Atomen im Alkyl steht, gegebenenfalls in einem Lösungsmittel und in Gegenwart einer anorganischen Säure, organischen Carbonsäure oder Sulfonsäure in an sich bekannter Weise umsetzt und gegebenenfalls einen erhaltenen Ester in die Carbonsäure und die erhaltene Verbindung der Formel (1) gegebenenfalls in ein therapeutisch verwendbares Ammonium-, Alkalimetall- oder Säureadditionssalz überführt, oder

b) – falls $R^1$ im Rahmen der für Formel (1) in Anspruch 1 genannten Bedeutungen eine zum N-Atom alpha-ständige $CH_2$-Gruppe aufweist – eine Acylaminoverbindung der Formel (4)

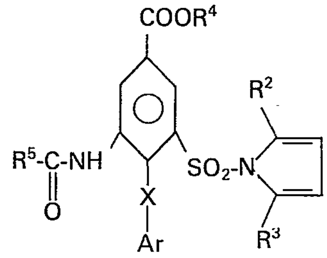

(4)

in der $R^2$, $R^3$, X und Ar die im Anspruch 1 angegebenen Bedeutungen haben, $R^4$ einen Alkylrest mit 1 bis 5 C-Atomen und $R^5$ einen Alkylrest mit 1 bis 4 C-Atomen, Phenyl, einen Furyl- oder einen Thienylrest bedeuten, mit einem Borhydrid in Gegenwart einer Lewis-Säure in an sich bekannter Weise reduziert und den erhaltenen Ester verseift und die erhaltene Verbindung gegebenenfalls in ein therapeutisch verwendbares Ammonium-, Alkalimetall- oder Säureadditionssalz überführt.

10. Verfahren nach Anspruch 9, dadurch gekennzeichnet, dass man Verbindungen der Formel (1) gemäss Anspruch 1 herstellt, in denen

- $R^1$ n-Butyl, Benzyl, 3-Thienylmethyl, 2-Thienylmethyl, 3-Furylmethyl, 2-Furylmethyl,
- $R^2$ Methyl oder Ethyl,
- $R^3$ Methyl, Ethyl oder n-Propyl,
- X ein Schwefelatom, Sauerstoffatom oder eine NH-Gruppe und
- Ar einen Phenylrest bedeuten

und ihre therapeutisch verwendbaren Ammonium- und Alkalimetallsalze.

11. Verfahren nach Anspruch 9, dadurch gekennzeichnet, dass man eine Verbindung herstellt, in der $R^1$ 3-Thienylmethyl, $R^2$ und $R^3$ Ethyl, X ein Sauerstoffatom und Ar Phenyl bedeuten.

12. Verfahren nach Anspruch 9, dadurch gekennzeichnet, dass man eine Verbindung herstellt, in der $R^1$ 3-Thienylmethyl, $R^2$ und $R^3$ Methyl, X eine NH-Gruppe und Ar Phenyl bedeuten.

13. Verfahren nach Anspruch 9, dadurch gekennzeichnet, dass man eine Verbindung herstellt, in der $R^1$ Benzyl, $R^2$ und $R^3$ Methyl, X ein Sauerstoffatom und Ar Phenyl bedeuten.

14. Verfahren nach Anspruch 9, dadurch gekennzeichnet, dass man eine Verbindung herstellt, in der $R^1$ 2-Thienylmethyl, $R^2$ und $R^3$ Methyl, X ein Sauerstoffatom und Ar Phenyl bedeuten.

15. Verfahren nach Anspruch 9, dadurch gekennzeichnet, dass man eine Verbindung herstellt, in der $R^1$ 3-Thienylmethyl, $R^2$ Methyl, $R^3$ Ethyl, X ein Schwefelatom und Ar Phenyl bedeuten.

16. Verfahren nach Anspruch 9, dadurch gekennzeichnet, dass man eine Verbindung herstellt, in der $R^1$ 3-Thienylmethyl, $R^2$ Methyl, $R^3$ Ethyl, X ein Sauerstoffatom und Ar Phenyl bedeuten.

17. Therapeutisches Mittel, enthaltend neben üblichen Träger- und Verdünnungsmitteln eine Verbindung nach Anspruch 1 als Wirkstoff.

18. Verbindungen nach Anspruch 1 zur Verwendung als Diuretika.

**Claims**

1. A compound of the formula (1)

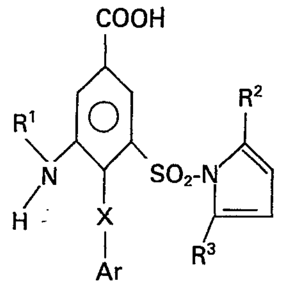

(1)

where
$R^1$ is alkyl of 2 to 5 carbon atoms, allyl, benzyl, furylmethyl or thienylmethyl,
$R^2$ and $R^3$ are each hydrogen, or
$R^2$ is methyl or ethyl, and
$R^3$ is methyl, ethyl or n-propyl,
Y is sulfur, oxygen or NH, and
Ar is phenyl, optionally substituted by chlorine,
and its therapeutically useful ammonium, alkali metal or acid addition salts.

2. A compound as claimed in claim 1, in which
$R^1$ is n-butyl, benzyl, 3-thienylmethyl, 2-thienylmethyl, 3-furylmethyl or 2-furylmethyl,
$R^2$ is methyl or ethyl,
$R^3$ is methyl, ethyl or n-propyl,
X is sulfor, oxygen or NH, and
Ar is phenyl,
and its therapeutically useful ammonium and alkali metal salts.

3. A compound as claimed in claim 1, in which $R^1$ is 3-thienylmethyl, $R^2$ and $R^3$ are each ethyl, X is oxygen and Ar is phenyl.

4. A compound as claimed in claim 1, in which $R^1$ is 3-thienylmethyl, $R^2$ and $R^3$ are each methyl, X is NH and Ar is phenyl.

5. A compound as claimed in claim 1, in which $R^1$ is benzyl, $R^2$ and $R^3$ are each methyl, X is oxygen and Ar is phenyl.

6. A compound as claimed in claim 1, in which $R^1$ is 2-thienylmethyl, $R^2$ and $R^3$ are each methyl, X is oxygen and Ar is phenyl.

7. A compound as claimed in claim 1, in which $R^1$ is 3-thienylmethyl, $R^2$ is methyl, $R^3$ is ethyl, X is sulfur and Ar is phenyl.

8. A compound as claimed in claim 1, in which $R^1$ is 3-thienylmethyl, $R^2$ is methyl, $R^3$ is ethyl, X is oxygen and Ar is phenyl.

9. A process for the preparation of a compound as claimed in claim 1, wherein

(a) a compound of the formula (2)

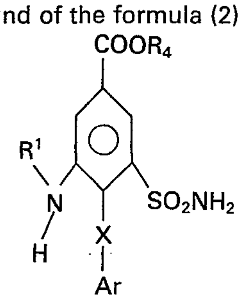

(2)

where $R^1$, X and Ar have the meanings given in claim 1, and $R^4$ is hydrogen or alkyl of 1 to 5 carbon atoms, is reacted in a conventional manner with a compound of the formula (3)

$$R^2\text{-CO-CH}_2\text{-CH}_2\text{-CO-}R^3$$
(3)

where $R^2$ and $R^3$ have the meanings given in claim 1, or, if $R^2$ and $R^3$ are each hydrogen, advantageously with a compound of the formula (3a)

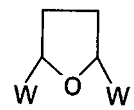

(3a)

where W is chlorine, alkoxy where alkyl is of 1 to 5 carbon atoms, or alkanoyloxy where alkyl is of 1 to 5 carbon atoms, in the presence or absence of a solvent and in the presence of an inorganic acid, organic carboxylic acid or sulfonic acid, and, if desired, a resulting ester is converted into the carboxylic acid, and the resulting compound of the formula (1) is, if desired, converted into a therapeutically useful ammonium, alkali metal or acid addition salt, or

(b) – if $R^1$, within the scope of the meanings given for formula (1) in claim, contains a $CH_2$ group alpha to the N atom – an acylamino compound of the formula (4)

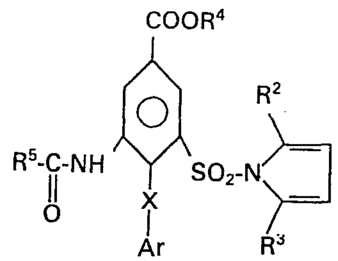

(4)

where $R^2$, $R^3$, X and Ar have the meanings given in claim 1, $R^4$ is alkyl of 1 to 5 carbon atoms, and $R^5$ is alkyl of 1 to 4 carbon atoms, phenyl, furyl or thienyl, is reduced in a conventional manner with a boron hydride in the presence of a Lewis acid, and the resulting ester is hydrolyzed, and the resulting compound is, if desired, converted into a therapeutically useful ammonium, alkali metal or acid addition salt.

10. A process as claimed in claim 9, wherein a compound of the formula (1) according to claim 1 is prepared, in which
$R^1$ is n-butyl, benzyl, 3-thienylmethyl, 2-thienylmethyl, 3-furylmethyl or 2-furylmethyl,
$R^2$ is methyl or ethyl,
$R^3$ is methyl, ethyl or n-propyl,
X is sulfur, oxygen, or NH, and
Ar is phenyl,
or its therapeutically useful ammonium and alkali metal salts.

11. A process as claimed in claim 9, wherein a compound is prepared, in which $R^1$ is 3-thienylmethyl, $R^2$ and $R^3$ are each ethyl, X is oxygen and Ar is phenyl.

12. A process as claimed in claim 9, wherein a compound is prepared, in which $R^1$ is 3-thienylmethyl, $R^2$ and $R^3$ are each methyl, X is NH and Ar is phenyl.

13. A process as claimed in claim 9, wherein a compound is prepared, in which $R^1$ is benzyl, $R^2$ and $R^3$ are each methyl, X is oxygen and Ar is phenyl.

14. A process as claimed in claim 9, wherein a compound is prepared, in which $R^1$ is 2-thienylmethyl, $R^2$ and $R^3$ are each methyl, X is oxygen and Ar is phenyl.

15. A process as claimed in claim 9, wherein a compound is prepared, in which $R^1$ is 3-thienylmethyl, $R^2$ is methyl, $R^3$ is ethyl, X is sulfur and Ar is phenyl.

16. A process as claimed in claim 9, wherein a compound is prepared, in which $R^1$ is 3-thienylmethyl, $R^2$ is methyl, $R^3$ is ethyl, X is oxygen and Ar is phenyl.

17. A therapeutic agent containing a compound as claimed in claim 1 as active ingredient, in addition to conventional carriers and diluents.

18. Compounds as claimed in claim 1 for use as diuretics.

**Revendications**

1. Composés de formule (1)

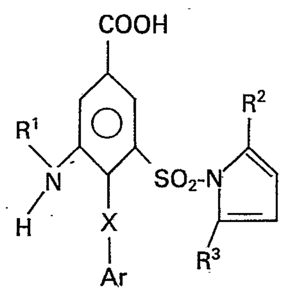

(1)

dans laquelle

$R^1$ représente un groupe alkyle en C 2-C 5, un groupe allyle, un groupe benzyle, un groupe furylméthyle ou un groupe thiénylméthyle,

$R^2$ et $R^3$ représentent des atomes d'hydrogène ou bien

$R^2$ représente un groupe méthyle ou éthyle et

$R^3$ représente un groupe méthyle, éthyle ou n-propyle,

X représente un atome de soufre ou d'oxygène ou un groupe NH et

Ar représente un groupe phényle éventuellement substitué par le chlore,

et leurs sels d'ammonium, de métaux alcalins ou formés par addition avec des acides, utilisables en thérapeutique.

2. Composés selon la revendication 1, dans lesquels

$R^1$ représente un groupe n-butyle, benzyle, 3-thiénylméthyle, 2-thiénylméthyle, 3-furylméthyle ou 2-furylméthyle,

$R^2$ représente un groupe méthyle ou éthyle,

$R^3$ représente un groupe méthyle, éthyle ou n-propyle,

X représente un atome de soufre, un atome d'oxygène ou un groupe NH et

Ar représente un reste phényle,

et leurs sels d'ammonium et de métaux alcalins utilisables en thérapeutique.

3. Composé selon la revendication 1, dans lequel $R^1$ représente un groupe 3-thiénylméthyle, $R^2$ et $R^3$ des groupes éthyle, X un atome d'oxygène et Ar un groupe phényle.

4. Composé selon la revendication 1, dans lequel $R^1$ représente un groupe 3-thiénylméthyle, $R^2$ et $R^3$ des groupes méthyle, X en groupe NH et Ar un groupe phényle.

5. Composé selon la revendication 1, dans lequel $R^1$ représente un groupe benzyle, $R^2$ et $R^3$ des groupes méthyle, X un atome d'oxygène et Ar un groupe phényle.

6. Composé selon la revendication 1, dans lequel $R^1$ représente un groupe 2-thiénylméthyle, $R^2$ et $R^3$ des groupes méthyle, X un atome d'oxygène et Ar un groupe phényle.

7. Composé selon la revendication 1, dans lequel $R^1$ représente un groupe 3-thiénylméthyle, $R^2$ un groupe méthyle, $R^3$ un groupe éthyle, X un atome de soufre et Ar un groupe phényle.

8. Composé selon la revendication 1, dans lequel $R^1$ représente un groupe 3-thiénylméthyle, $R^2$ un groupe méthyle, $R^3$ un groupe éthyle, X un atome d'oxygène et Ar un groupe phényle.

9. Procédé de préparation des composés selon la revendication 1, caractérisé en ce que:

a) on fait réagir, de manière connue en soi, un composé de formule (2)

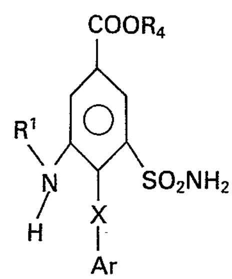

(2)

dans laquelle $R^1$, X et Ar ont les significations indiquées dans la revendication 1 et $R^4$ représente un atome d'hydrogène ou un groupe alkyle en C 1-C 5, avec un copmposé de formule (3)

$$R^2\text{-CO-CH}_2\text{-CH}_2\text{-CO-}R^3$$

(3)

dans laquelle $R^2$ et $R^3$ ont les significations indiquées dans la revendication 1, ou bien, lorsque $R^2$ et $R^3$ représentent des atomes d'hydrogène, de préférence avec un composé de formule (3a)

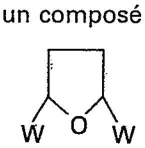

(3a)

dans laquelle W représente un atome de chlore, un groupe alcoxy contenant 1 à 5 atomes de

carbone dans la partie alkyle ou un groupe alcanoyloxy contenant 1 à 5 atomes de carbone dans la partie alkyle, éventuellement dans un solvant et en présence d'un acide minéral, d'un acide organique carboxylique ou sulfonique, et le cas échéant on convertit un ester obtenu en l'acide carboxylique et le cas échéant on convertit le composé de formule 1 ainsi obtenu en un sel d'ammonium, de métal alcalin, ou formé par addition avec un acide, utilisabe en thérapeutique, ou bien

b) – lorsque $R^1$, dans le cadre des définitions indiquées pour la formule 1 dans la revendication 1, contient un groupe $CH_2$ en position alpha de l'atome d'azote – on réduit de manière connue en soi un composé acylaminé de formule (4)

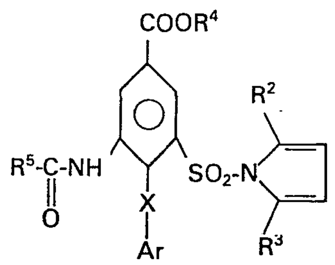

(4)

dans laquelle $R_2$, $R^3$, X et Ar ont les significations indiquées dans la revendication 1, $R^4$ représente un groupe alkyle en C 1-C 5 et $R^5$ un groupe alkyle en C 1-C 4, un groupe phényle, furyle ou thiényle, à l'aide d'un borohydrure en présence d'un acide de Lewis et on saponifie l'ester obtenu, et le cas échéant on convertit le composé obtenu en un sel d'ammonium, de métal alcalin ou formé par addition avec un acide, utilisable en thérapeutique.

10. Procédé selon la revendication 9, caractérisé en ce que l'on prépare des composés de formule 1 selon la revendication 1 dans lesquels

$R^1$ représente un groupe n-butyle, benzyle, 3-thiénylméthyle, 2-thiénylméthyle, 3-furyméthyle, 2-furylméthyle,

$R^2$ représente un groupe méthyle ou éthyle,

$R^3$ représente un groupe méthyle, éthyle ou n-propyle,

X représente un atome de soufre, un atome d'oxygène ou un groupe NH et

Ar représente un groupe phényle

et leurs sels d'ammonium et de métaux alcalins utilisables en thérapeutique.

11. Procédé selon la revendication 9, caractérisé en ce que l'on prépare un composé dans lequel $R^1$ représente un groupe 3-thiénylméthyle, $R^2$ et $R^3$ des groupes éthyle, X un atome d'oxygène et Ar un groupe phényle.

12. Procédé selon la revendication 9, caractérisé en ce que l'on prépare un composé dans lequel $R^1$ représente un groupe 3-thiénylméthyle, $R^2$ et $R^3$ des groupes méthyle, X un groupe NH et Ar un groupe phényle.

13. Procédé selon la revendication 9, caractérisé en ce que l'on prépare un composé dans lequel $R^1$ représente un groupe benzyle, $R^2$ et $R^3$ des groupes méthyle, X un atome d'oxygène et Ar un groupe phényle.

14. Procédé selon la revendication 9, caractérisé en ce que l'on prépare un composé dans lequel $R^1$ représente un groupe 2-thiénylméthyle, $R^2$ et $R^3$ des groupes méthyle, X un atome d'oxygène et Ar un groupe phényle.

15. Procédé selon la revendication 9, caractérisé en ce que l'on prépare un composé dans lequel $R^1$ représente un groupe 3-thiénylméthyle, $R^2$ un groupe méthyle, $R^3$ un groupe éthyle, X un atome de soufre et Ar un groupe phényle.

16. Procédé selon la revendication 9, caractérisé en ce que l'on prépare un composé dans lequel $R^1$ représente un groupe 3-thiénylméthyle, $R^2$ un groupe méthyle, $R^3$ un groupe éthyle, X un atome d'oxygène et Ar un groupe phényle.

17. Agent thérapeutique contenant, avec des véhicules et diluants usuels, un composé selon la revendication 1 en tant que substance active.

18. Composés selon la revendication 1 pour l'utilisation en tant que diurétiques.